# EUROPEAN PATENT SPECIFICATION

(11) **EP 4 394 015 B1**
(45) Date of publication and mention of the grant of the patent: **22.04.2026**
(21) Application number: 21969501.2
(22) Date of filing: 29.12.2021
(51) Int. Cl.: C09K 11/02, C09K 11/88

(54) **QUANTUM DOT LIGAND, PREPARATION METHOD FOR QUANTUM DOT FILM LAYER, AND QUANTUM DOT LIGHT EMITTING DEVICE**
QUANTENPUNKTLIGAND, HERSTELLUNGSVERFAHREN FÜR QUANTENPUNKTFILMSCHICHT UND QUANTENPUNKTLICHTEMITTIERENDE VORRICHTUNG
LIGAND À POINTS QUANTIQUES, PROCÉDÉ DE PRÉPARATION DE COUCHE DE FILM À POINTS QUANTIQUES ET DISPOSITIF ÉLECTROLUMINESCENT À POINTS QUANTIQUES

(43) Date of publication of application: 03.07.2024
(73) Proprietor: BOE Technology Group Co., Ltd., Chaoyang District, Beijing 100015 (CN); Beijing BOE Technology Development Co., Ltd., Beijing 100176 (CN)
(72) Inventor: CHEN, Zhuo, Beijing 100176 (CN)
(74) Representative: Potter Clarkson
(86) International application number: PCT/CN2021/142713
(87) International publication number: WO 2023/123133

(56) References cited:
- WO-A1-2021/164768
- CN-A- 110 590 549
- CN-A- 111 909 683
- CN-A- 112 300 784
- CN-A- 113 088 279
- KR-A- 20190 047 573
- US-A1- 2010 117 110
- US-A1- 2019 312 204
- TAKAOKA K ET AL: "Synthesis and photoreactivity of caged blockers for glutamate transporters", BIOORGANIC & MEDICINAL CHEMISTRY LETTERS, ELSEVIER, AMSTERDAM NL, vol. 13, 1 January 2003 (2003-01-01), pages 965 - 970, XP002266315, ISSN: 0960-894X, DOI: 10.1016/S0960-894X(02)01042-9

## Description

### TECHNICAL FIELD

The present disclosure relates to the field of display technology, and in particular relates to a quantum dot ligand, a preparation method for quantum dot film layer and a quantum dot light emitting device.

### BACKGROUND

With the in-depth development of quantum dot preparation technology, the stability of quantum dots as well as the luminescence efficiency is constantly improving. The research of Quantum Dot Light Emitting Diodes (QLED) is constantly deepening, and the prospect of QLED application in the field of display becomes increasingly bright. However, the efficiency of QLED has not yet reached the level of mass production. One of the major reasons is that the high-resolution patterning technology of QLED has not yet made a breakthrough.

The inorganic nanoparticle characteristics of quantum dots prevent them from being formed into a film by evaporation and patterned; it is difficult to achieve a high resolution by the inkjet printing method.

The above information disclosed in the background section is intended only to enhance the understanding of the background of the present disclosure, and therefore it may include information that does not constitute prior art known to those of ordinary skill in the art.

WO 2021/164768 A1 discloses a cross-linking ligand, a method for patterning a nanoparticle layer, a quantum dot light-emitting device and a display device. The cross-linking ligand comprises at least two coordinating groups, and at least one photosensitive degradable group and at least one thermosensitive crosslinking group are linked between the coordinating groups. The method for patterning a nanoparticle layer comprises: forming a nanoparticle layer on a substrate; attaching a solution containing the cross-linking ligand to the substrate; the cross-linking ligand forming crosslinks between nanoparticles; performing light irradiation treatment on a pre-set region of the substrate to degrade the cross-linking ligand in the pre-set region, thereby removing crosslinks between nanoparticles; removing the nanoparticles in the pre-set region; and performing thermal treatment on the substrate, so that crosslinks are formed between thermosensitive cross-linking groups in the cross-linking ligand, and patterning is completed.

Takaoka K, et al. "Synthesis and photoreactivity of caged blockers for glutamate transporters", Bioorganic & Medicinal Chemistry Letters, Volume 13, Issue 5, 10 March 2003, pages 965-970 discloses a synthesis of alpha-CMCM-L-TBOA (1a) possessing [7-(carboxymethoxy)coumarin-4-yl]methyl ester as a caging group. alpha-CMCM-L-TBOA (1a) is biologically inactive until UV irradiation and the photolysis of 1a immediately released L-TBOA to show glutamate uptake inhibition.

### SUMMARY

The present invention comprises a quantum dot ligand, a quantum dot-ligand material, a method for preparing a quantum dot film layer and a method for preparing a quantum dot light-emitting device as defined by the claims. Embodiments that do not fall within the scope of the claims should be regarded as examples useful for understanding the present invention. It is an object of the present disclosure to provide a quantum dot ligand, a method for preparing a quantum dot film layer, and a quantum dot light-emitting device. The quantum dot ligand provides a basis for the quantum dot film layer to be able to be formed by a photolithography process.

In order to realize the above object, the present disclosure adopts the following technical scheme:

According to a first aspect of the present disclosure, there is provided a quantum dot ligand. The quantum dot ligand has a structural general formula as shown in Formula I,

X-Y-Z Formula I

;
where X is a coordination group for forming a coordination bond with the quantum dot body;
Z is a dissolving group, and the dissolving group is selected from a polar group;
Y includes a linking group and a photosensitive group connected with each other, and the linking group is connected between the coordination group and the photosensitive group;
the linking group is selected from an alkylene group;
the photosensitive group is capable of undergoing bond breaking under light condition such that the quantum dot ligand decomposes into a first unit including the coordination group and the linking group, and a second unit including the dissolving group, and polarity of the first unit is less than polarity of the second unit.

In some embodiments of the present disclosure, the positive and negative charge centers of the dissolving group do not overlap.

In some embodiments of the present disclosure, a dielectric constant of the first unit is less than a dielectric constant of the second unit.

In some embodiments of the present disclosure, the dissolving group has a dipole moment µ>0.5.

In some embodiments of the present disclosure, after the photosensitive group undergoes bond breaking under light conditions, amino, hydroxyl, or carboxyl groups are formed at the end of the first unit.

In some embodiments of the present disclosure, Y further includes a solubilizing group, the solubilizing group is connected between the photosensitive group and the dissolving group, and the solubilizing group is selected from a structure comprising 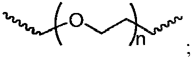
when Y further includes the solubilizing group, the second unit further includes the solubilizing group;
where n is any integer selected from 1 to 9.

In some embodiments of the present disclosure, the coordination group is selected from amino, carboxylic acid, sulfhydryl, phosphine or phosphine oxide groups.

In some embodiments of the present disclosure, the dissolving group is selected from a quaternary ammonium salt group.

In some embodiments of the present disclosure, the dissolving group is selected from the structure of 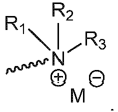
where R₁, R₂, and R₃ are same or different, which are independently selected from methyl group and ethyl group;
M is selected from halogen atoms.

In some embodiments of the present disclosure, the linking group is selected from an alkylene group with a carbon atom number of 2 to 8.

In some embodiments of the present disclosure, not falling within the scope of the claims, the photosensitive group is selected from the structure including

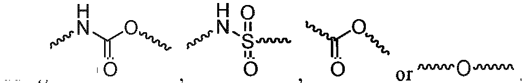

According to the invention, the photosensitive group is selected from a group including the following structures:

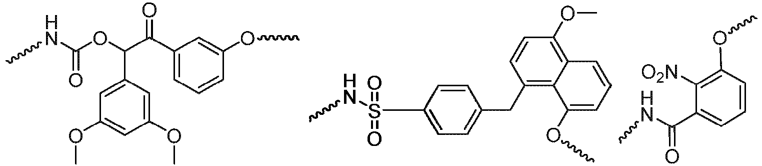

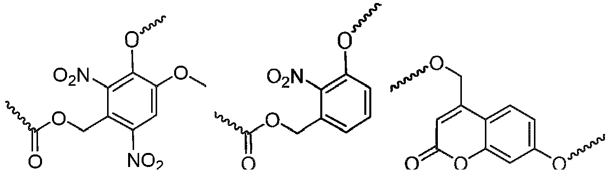

In some embodiments of the present disclosure, the quantum dot ligand is selected from a group including the following structures: 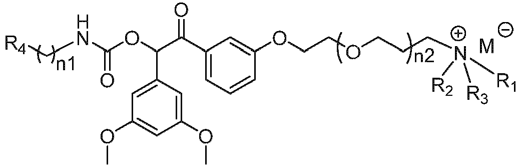 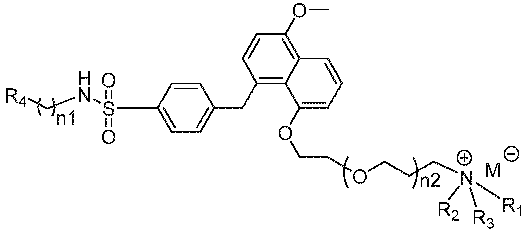 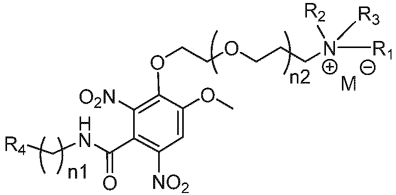 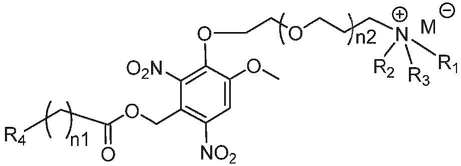 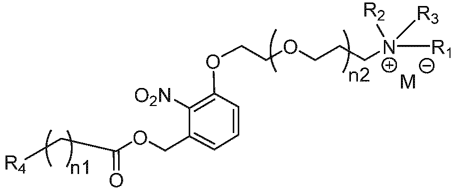 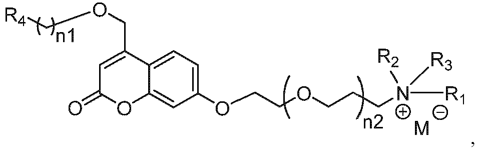
where R₁, R₂, and R₃ are same or different, and are independently selected from methyl group and ethyl group;
M is selected from chlorine or bromine;
R₄ is selected from amino, carboxylic acid, sulfhydryl, phosphine or phosphine oxide groups;
n1 is any integer selected from 2 to 8;
n2 is any integer selected from 1 to 9.

In some embodiments of the present disclosure, the quantum dot ligand is selected from a group including the following structures: 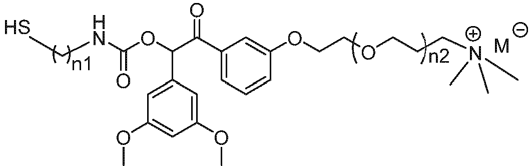 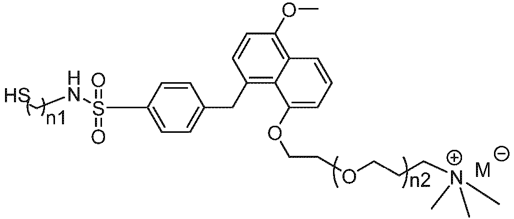 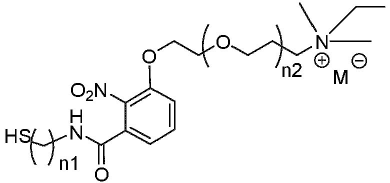 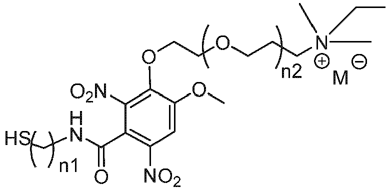 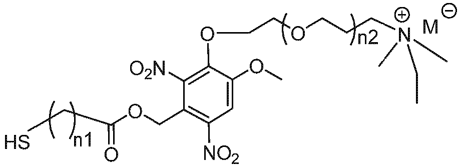 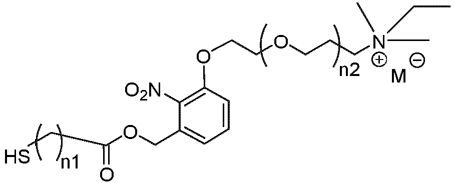 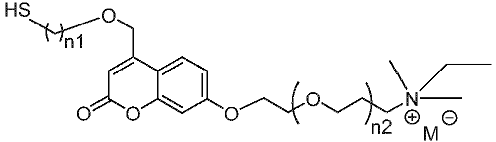
where M is selected from chlorine or bromine;
n1 is any integer selected from 2 to 8;
n2 is any integer selected from 1 to 9.

According to a second aspect of the present disclosure, there is provided a quantum dot-ligand material, including the quantum dot body and the quantum dot ligand according to the first aspect, the quantum dot ligand is connected to the quantum dot body through the coordination bond.

According to a third aspect of the present disclosure, there is provided a method for preparing a quantum dot film layer, including:
providing the quantum dot-ligand material according to the second aspect;
coating the quantum dot-ligand material on a substrate, and performing an exposure treatment such that the photosensitive group in the quantum dot-ligand material undergoes bond breaking to decompose the quantum dot-ligand material into a first material including the quantum dot body, the coordination group, and the linking group, as well as a second material including the dissolving group;
performing a development process, where the second material is soluble in the development solution, the first material is insoluble in the development solution, and the first material forms the quantum dot film layer.

In some embodiments of the present disclosure, when the quantum dot ligand further includes a solubilizing group, the second material further includes the solubilizing group.

According to a fourth aspect of the present disclosure, there is provided a method for preparing a quantum dot light-emitting device, including:
providing a first color quantum dot-ligand solution, where the first color quantum dot-ligand solution is a mixed solution including a first color quantum dot body and the quantum dot ligand according to the first aspect;
providing a second color quantum dot-ligand solution, where the second color quantum dot-ligand solution is a mixed solution including a second color quantum dot body and the quantum dot ligand according to the first aspect;
forming a first color sub-pixel by coating the first color quantum dot-ligand solution on a substrate and performing exposure development;
forming a second color sub-pixel by coating the second color quantum dot-ligand solution on the substrate and performing exposure development.

According to a fifth aspect of the present disclosure, there is provided a quantum dot light-emitting device, including a functional layer, where the functional layer includes a quantum dot film layer, and the quantum dot film layer includes the first unit according to the first aspect.

In some embodiments of the present disclosure, the quantum dot light-emitting device further includes an anode and a cathode, and the functional layer is located between the anode and the cathode.

In some embodiments of the present disclosure, the quantum dot light-emitting device further includes a light-emitting unit, with the quantum dot film layer located on one side of the light-emitting unit.

In some embodiments of the present disclosure, the quantum dot film layer includes a plurality of sub-units, the sub-units include a first color quantum dot and a second color quantum dot, a material of the first color quantum dot includes a first color quantum dot body and the first unit, and a material of the second color quantum dot comprises a second color quantum dot body and the quantum dot ligand in which no bond breaking occurs;
in at least one sub-unit of the plurality of sub-units, a mass ratio of the material of the second color quantum dot to the material of the first color quantum dot is less than 10⁻³.

According to a sixth aspect of the present disclosure, there is provided a display device, including the quantum dot light-emitting device according to the fifth aspect.

The present disclosure provides a quantum dot ligand including a coordination group, a photosensitive group, and a dissolving group. The coordination group is capable of forming a coordination bond with the quantum dot body; the dissolving group is selected from a polar group, which helps to enhance the solubility of the quantum dot ligand; and the photosensitive group is capable of breaking the bond under the light condition, which allows the quantum dot ligand to decompose into a first unit and a second unit. The first unit includes the coordination group and the linking group, and the second unit includes the dissolving group, and polarity of the first unit is less than polarity of the second unit, thereby providing a basis for the quantum dot film layer to be formed by a photolithographic process.

### BRIEF DESCRIPTION OF THE DRAWINGS

The foregoing and other features and advantages of the present disclosure will become more apparent by describing the exemplary embodiments thereof in detail with reference to the accompanying drawings.
FIG. 1 is a schematic diagram of a structure of a first color-quantum dot ligand solution coated on a substrate in an exemplary embodiment of the present disclosure;
FIG. 2 is a schematic diagram of a structure for forming a first color sub-pixel in an exemplary embodiment of the present disclosure;
FIG. 3 is a schematic diagram of a second color-quantum dot ligand solution structure coated on a substrate in an exemplary embodiment of the present disclosure;
FIG. 4 is a schematic diagram of a structure for forming a second color sub-pixel in an exemplary embodiment of the present disclosure;
FIG. 5 is a schematic diagram of a third color-quantum dot ligand solution structure coated on a substrate in an exemplary embodiment of the present disclosure;
FIG. 6 is a schematic diagram of a structure for forming a third color sub-pixel in an exemplary embodiment of the present disclosure;
FIG. 7 is a schematic diagram of the structure of a quantum dot light-emitting device in an exemplary embodiment of the present disclosure;
FIG. 8 is a schematic diagram of the structure of a quantum dot light-emitting device in another exemplary embodiment of the present disclosure.

### DETAILED DESCRIPTION

Example embodiments will now be described more fully with reference to the accompanying drawings. However, the example embodiments are capable of being implemented in a variety of forms and should not be construed as being limited to the examples set forth herein; rather, the provision of these embodiments allows for the present disclosure to be more comprehensive and complete and conveys the idea of the example embodiments in a comprehensive manner to those skilled in the art. The described features, structures, or characteristics may be combined in one or more embodiments in any suitable manner. In the following description, many specific details are provided thereby giving a full understanding of the embodiments of the present disclosure.

In the drawings, the thickness of regions and layers may be exaggerated for clarity. The same reference numbers in the drawings denote the same or similar structures, and thus their detailed descriptions will be omitted.

The described features, structures or characteristics may be combined in one or more embodiments in any suitable manner. In the following description, many specific details are provided thereby giving a full understanding of the embodiments of the present disclosure. However, those skilled in the art will realize that it is possible to practice the technical solutions of the present disclosure without one or more of the particular details described, or that other methods, components, materials, etc. may be employed. In other cases, well-known structures, materials, or operations are not shown or described in detail to avoid obscuring the main technical ideas of the present disclosure.

When a structure is "on" another structure, it may mean that a structure is integrally formed on another structure, or that a structure is "directly" provided on another structure, or that a structure is "indirectly" provided on another structure through other structure.

The terms "a", "one", and "said" are used to indicate the presence of one or more elements/components/etc.; the terms "including" and "having" are used to indicate open-ended inclusion, and mean that there may be additional elements/components/etc. in addition to those listed. The terms "first", "second", etc. are used only as markers and not as quantitative limitations on their objects.

Quantum Dots (QDs) are composed of zinc, cadmium, selenium and sulfur atoms, which are nano-materials with crystal diameters between 2-10 nm. They have unique optoelectronic properties, and when stimulated by photo-electricity, they will emit a variety of pure monochromatic light of different colors according to the size of the diameter of the quantum dots, and are able to change the color of light from the light source.

Quantum dots are generally used to form quantum dot film layers for quantum dot light emitting devices. The quantum dot light emitting device generally includes an anode, a hole transport layer, a quantum dot film layer, an electron transport layer, and a cathode which are stacked in sequence. When a voltage is applied to the cathode and the anode, the two electrodes generate an electric field. Under the action of the electric field, the electrons on the cathode side move towards the quantum dot film layer, and the holes on the anode side move toward the light-emitting layer. The electrons and the holes combine to form excitons in the quantum dot film layer. The excitons are in excited state and release energy outwards, which causes the quantum dot film layer to emit light externally.

In the related art, due to the disadvantage of being susceptible to heat and moisture, the same evaporation manner as the self-luminous OLED cannot be achieved for the quantum dots. The quantum dots can only be inkjet printed at present. However, it is difficult to achieve a high resolution by inkjet printing.

The present disclosure provides a quantum dot ligand, and a method for preparing and forming a quantum dot film layer by using the quantum dot ligand. The quantum dot ligand has photosensitive property, and directly forms red, green, and blue sub-pixels through photolithography when preparing the quantum dot film layer, bypassing technical difficulties such as improving resolution in inkjet printing, such as the need for a higher-precision printing nozzle. The quantum dot ligand provided in the present disclosure helps to realize the production of high-resolution QLED products, facilitates the preparation of process aspects, improves process yields, and can significantly increase the utilization rate of quantum dot materials, thereby providing a basis for the large-scale industrialization of QLEDs.

The present disclosure provides a quantum dot ligand. The quantum dot ligand has a structural general formula as shown in Formula I,

X-Y-Z Formula I;

where X is a coordination group for forming a coordination bond with the quantum dot body;
Z is a dissolving group, and the dissolving group is selected from a polar group;
Y includes a linking group and a photosensitive group connected with each other, and the linking group is connected between the coordination group and the photosensitive group;
the linking group is selected from an alkylene group;
the photosensitive group is capable of undergoing bond breaking under light condition such that the quantum dot ligand decomposes into a first unit including the coordination group and the linking group, and a second unit including the dissolving group. The polarity of the first unit is less than the polarity of the second unit.

The quantum dot ligand, provided by the present disclosure, includes a coordination group, a photosensitive group, and a dissolving group. The coordination group can form a coordination bond with the quantum dot body. The dissolving group is selected from a polar group, which helps to enhance the solubility of the quantum dot ligand. The photosensitive group can break the bond under the light condition, such that the quantum dot ligand decomposes into a first unit and a second unit. The first unit includes the coordination group and the linking group, and the second unit includes the dissolving group. The polarity of the first unit is less than the polarity of the second unit, thus providing a basis for the quantum dot film layer to be formed by a photolithographic process.

Quantum dots (QDs) are inorganic semiconductor nanoparticles synthesized by a solution method and having a size between 1 nm and 10 nm. The size is similar to or less than the exciton Bohr radius of the particle. Quantum dots are prone to agglomeration due to their small sizes and large specific surface area. Quantum dots have many surface defects, so the surface of quantum dots is usually coated with an organic surface ligand during application. The organic surface ligand not only provides protection, but also enhances the solubility of the quantum dots in solution. The migration of charge carriers (electrons and holes) in the quantum dots is restricted within the interior of the quantum dots, which gives them unique optical and electrical properties. Due to the unique size-dependent property, the light-absorbing and light-emitting properties of the quantum dots can be easily adjusted by controlling size, shape, or surface structure of the particle.

The quantum dot body of the present disclosure may be a semiconductor nanocrystal and may have a variety of shapes such as spherical, conical, dobby, and/or cubic nanoparticles, nanotubes, nanowires, nanofibers, nanoplate particles, quantum rods, or quantum sheets. Herein, a quantum rod may be a quantum dot body having an aspect ratio (length-to-diameter ratio) (length: width ratio) of greater than about 1, for example greater than or equal to about 2, greater than or equal to about 3, or greater than or equal to about 5. For example, the quantum rod may have an aspect ratio less than or equal to about 50, less than or equal to about 30, or less than or equal to about 20.

The quantum dot body may have, for example, a particle diameter (average maximum particle length for non-spherical shapes) of, e.g., from about 1 nm to about 100 nm, from about 1 nm to about 80 nm, from about 1 nm to about 50 nm, or from about 1 nm to about 20 nm.

The energy band gap of the quantum dot body may be controlled according to the size and composition of the quantum dot body, and the luminescence wavelength may accordingly be controlled. For example, when the size of the quantum dot body increases, the quantum dot body may have a narrow energy band gap and is therefore configured to emit light in a relatively long wavelength region, whereas when the size of the quantum dot body decreases, the quantum dot body may have a wide energy band gap and is therefore configured to emit light in a relatively short wavelength region. For example, the quantum dot body may be configured to emit light in a predetermined wavelength region in the visible region depending on its size and/or composition. For example, the quantum dot body may be configured to emit a second color light, a third color light, or a first color light. The second color light may have, for example, a peak emission wavelength (λ max) in the range of about 430 nm to about 480 nm. The third color light may have, for example, a peak emission wavelength (λ max) in the range of about 600 nm to about 650 nm. The first color light may have, for example, a peak emission wavelength (λ max) in the range of about 520 nm to about 560 nm. However, the present disclosure is not limited to this.

For example, the average particle size of the quantum dot body configured to emit the second color light may be, for example, less than or equal to about 4.5nm, and for example, less than or equal to about 4.3nm, less than or equal to about 4.2nm, less than or equal to about 4.1nm, or less than or equal to about 4.0nm. Within the range, the average particle size of the quantum dot body can be from about 2.0nm to about 4.5nm, for example, from about 2.0nm to about 4.3nm, from about 2.0nm to about 4.2nm, from about 2.0nm to about 4.1nm, or from about 2.0nm to about 4.0nm.

The quantum dot body may have a quantum yield of, for example, greater than or equal to about 10%, greater than or equal to about 20%, greater than or equal to about 30%, greater than or equal to about 50%, greater than or equal to about 60%, greater than or equal to about 70%, or greater than or equal to about 90%.

The quantum dot body may have a relatively narrow half width (FWHM). Here, FWHM is half the wavelength width corresponding to the peak absorption point. When FWHM is narrow, it can be configured to emit light in a narrow wavelength region and achieve high color purity. The quantum dot body may have FWHM of, for example, less than or equal to about 50nm, less than or equal to about 49nm, less than or equal to about 48nm, less than or equal to about 47nm, less than or equal to about 46nm, less than or equal to about 45nm, less than or equal to about 44nm, less than or equal to about 43nm, less than or equal to about 42nm, less than or equal to about 41nm, less than or equal to about 40nm, less than or equal to about 39nm, less than or equal to about 38nm, less than or equal to about 37nm, less than or equal to about 36nm, less than or equal to about 35nm, less than or equal to about 34nm, less than or equal to about 33nm, less than or equal to about 32nm, less than or equal to about 31mn, less than or equal to about 30nm, less than or equal to about 29nm, or less than or equal to about 28nm. Within the range, it may have FWHM of, for example, about 2nm to about 49nm, about 2nm to about 48nm, about 2nm to about 47nm, about 2nm to about 46nm, about 2nm to about 45nm, about 2nm to about 44nm, about 2nm to about 43nm, about 2nm to about 42nm, about 2nm to about 41nm, about 2nm to about 40nm, about 2nm to about 39nm, about 2nm to about 38nm, about 2nm to about 37nm, about 2nm to about 36nm, about 2nm to about 35nm, about 2nm to about 34nm, about 2nm to about 33nm, about 2nm to about 32nm, about 2nm to about 31nm, about 2nm to about 30nm, about 2nm to about 29nm, or about 2nm to about 28nm.

For example, the quantum dot body may include II-VI semiconductor compound, III-V semiconductor compound, IV-VI semiconductor compound, IV semiconductor compound, I-III-VI semiconductor compound, I-II-IV-VI semiconductor compound, II-III-V semiconductor compound, or combinations thereof. The II-VI semiconductor compound may be selected from binary compound such as CdSe, CdTe, ZnS, ZnSe, ZnTe, ZnO, HgS, HgSe, HgTe, MgSe, MgS, or mixtures thereof; ternary compound such as CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, MgZnSe, MgZnS, or mixtures thereof; and quaternary compound such as HgZnTeS, CdZnSeS, CdZnSeTe, CdZnSTe, CdHgSeS, CdHgSeTe, CdHgSTe, HgZnSeS, HgZnSeTe, HgZnSTe, or mixtures thereof, but is not limited to this. The III-V semiconductor compound may be selected from binary compound such as GaN, GaP, GaAs, GaSb, AlN, AlP, AlAs, AlSb, InN, InP, InAs, InSb, or mixtures thereof; ternary compound such as GaNP, GaNAs, GaNSb, GaPAs, GaPSb, AlNP, AlNAs, AlNSb, AlPAs, AlPSb, InNP, InNAs, InNSb, InPAs, InPSb, or mixtures thereof; and quaternary compound such as GaAlNP, GaAINAs, GaAlNSb, GaAlPAs, GaAlPSb, GaInNP, GaInNAs, GaInNSb, GaInPAs, GaInPSb, InAlNP, InAlNAs, InAlNSb, InAlPAs, InAlPSb, or mixtures thereof, but is not limited to this. The IV-VI semiconductor compound can be selected from, for example, binary compound such as SnS, SnSe, SnTe, PbS, PbSe, PbTe, or mixtures thereof; ternary compound such as SnSeS, SnSeTe, SnSTe, PbSeS, PbSeTe, PbSTe, SnPbS, SnPbSe, SnPbTe, or mixtures thereof; and quaternary compound such as SnPbSSe, SnPbSeTe, SnPbSTe, or mixtures thereof, but is not limited to this. The IV group semiconductor can be selected from, for example, monoatomic (unary) semiconductor such as Si, Ge, or mixtures thereof; and binary semiconductor compound such as SiC, SiGe, and mixtures thereof, but is not limited to this. The I-III-VI semiconductor compound may be, for example, CuInSe2, CuInS2, CuInGaSe, CuInGaS, or mixtures thereof, but is not limited to this. The I-II-IV-VI semiconductor compound may be, for example, CuZnSnSe, CuZnSnS, or mixtures thereof, but is not limited to this. The II-III-V semiconductor compound may include InZnP. but is not limited to this.

The quantum dot body can include the monoatomic semiconductor, the binary semiconductor compound, the ternary semiconductor compound, or the quaternary semiconductor compound in a basically uniform concentration or locally different concentration distribution.

For example, the quantum dot body may include a cadmium (Cd) free quantum dot body. The cadmium free quantum dot body is a quantum dot body that does not include cadmium (Cd). Cadmium (Cd) can cause serious environmental/health problems and is a restricted element under the Restriction of Hazardous Substances (RoHS) Directive in many countries, and therefore non cadmium based quantum dot body can be effectively used.

As an example, the quantum dot body can be a semiconductor compound including zinc (Zn), as well as at least one of tellurium (Te), and selenium (Se). For example, the quantum dot body can be a Zn-Te semiconductor compound, a Zn-Se semiconductor compound, and/or a Zn-Te-Se semiconductor compound. For example, the amount of tellurium (Te) in the Zn-Te-Se semiconductor compound may be less than the amount of selenium (Se). The semiconductor compound may have a peak emission wavelength in a wavelength range of less than or equal to about 480nm, for example from about 430nm to about 480nm (λ Max), and can be configured to emit the second color light.

For example, the quantum dot body may be a semiconductor compound including indium (In), as well as at least one of zinc (Zn) and phosphorus (P). For example, the quantum dot body can be an In-P semiconductor compound and/or an In-Zn-P semiconductor compound. For example, in the In-Zn-P semiconductor compound, the molar ratio of zinc (Zn) to indium (In) can be greater than or equal to about 25. The semiconductor compound may have a peak emission wavelength in a wavelength range of less than about 700nm, for example from about 600nm to about 650nm (λ Max), and can be configured to emit the third color light.

The quantum dot body may have a core-shell structure, where one quantum dot body surrounds another quantum dot body. For example, the core and shell of the quantum dot body may have an interface, and elements of at least one of the core or shell in the interface may have a concentration gradient, where the concentration of elements of the shell decreases towards the core. For example, the material composition of the shell of the quantum dot body has a higher energy band gap than the material composition of the core of the quantum dot body, and thus the quantum dot body can exhibit quantum confinement effect.

The quantum dot body may have a quantum dot core and multiple layers of quantum dot shells surrounding the core. Here, the multi-layer shells have at least two shells, each of which can be of a single composition, alloy, and/or have a concentration gradient.

For example, a shell of the multi-layer shells far away from the core may have a higher energy band gap than a shell of the multi-layer shells close to the core, and thus the quantum dot body may exhibit quantum confinement effect.

For example, the quantum dot body with the core-shell structure may include, for example, a core, where the core includes a first semiconductor compound, and the first semiconductor compound includes zinc (Zn), as well as at least one of tellurium (Te) and selenium (Se); and a shell provided on at least a portion of the core and including a fourth semiconductor compound with a composition different from that of the core.

For example, the first semiconductor compound may be a Zn-Te-Se based semiconductor compound including zinc (Zn), tellurium (Te), and selenium (Se), for example, a Zn-Se based semiconductor compound including a small amount of tellurium (Te), for example, a semiconductor compound represented by ZnTexSe1-x, where x is greater than about 0 and less than or equal to 0.05.

For example, in the first semiconductor compound based on Zn-Te-Se, the molar amount of zinc (Zn) can be higher than that of selenium (Se), and the molar amount of selenium (Se) can be higher than that of tellurium (Te). For example, in the first semiconductor compound, the molar ratio of tellurium (Te) to selenium (Se) may be less than or equal to about 0.05, less than or equal to about 0.049, less than or equal to about 0.048, less than or equal to about 0.047, less than or equal to about 0.045, less than or equal to about 0.044, less than or equal to about 0.043, less than or equal to about 0.042, less than or equal to about 0.041, less than or equal to about 0.04, less than or equal to about 0.039, less than or equal to about 0.035, less than or equal to about 0.03, less than or equal to about 0.029, less than or equal to about 0.025, less than or equal to about 0.024, less than or equal to about 0.023, less than or equal to about 0.022, less than or equal to about 0.021, less than or equal to about 0.02, less than or equal to about 0.019, less than or equal to about 0.018, less than or equal to about 0.017, less than or equal to about 0.016, less than or equal to about 0.015, less than or equal to about 0.014, less than or equal to about 0.013, less than or equal to about 0.012, less than or equal to about 0.011, or less than or equal to about 0.01. For example, in the first semiconductor compound, the molar ratio of tellurium (Te) to zinc (Zn) may be less than or equal to about 0.02, less than or equal to about 0.019, less than or equal to about 0.018, less than or equal to about 0.017, less than or equal to about 0.016, less than or equal to about 0.015, less than or equal to about 0.014, less than or equal to about 0.013, less than or equal to about 0.012, less than or equal to about 0.011, or less than or equal to about 0.010.

The second semiconductor compound may include, for example, II-VI semiconductor compound, III-V semiconductor compound, IV-VI semiconductor compound, IV semiconductor, I-III-VI semiconductor compound, I-II-IV-VI semiconductor compound, II-III-V semiconductor compound, or combinations thereof. The examples of II-VI semiconductor compound, III-V semiconductor compound, IV-VI semiconductor compound, IV semiconductor, I-III-VI semiconductor compound, I-II-IV-VI semiconductor compound, and II-III-V semiconductor compound are the same as described above.

For example, the second semiconductor compound may include zinc (Zn), selenium (Se), and/or sulfur (S). For example, the shell may include ZnSeS, ZnSe, ZnS, or a combination thereof. For example, the shell may include at least one inner shell provided near the core and the outermost shell provided on the outermost side of the quantum dot body. The inner shell may include ZnSeS, ZnSe, or a combination thereof, and the outermost shell may include ZnS. For example, the shell may have a concentration gradient of one component. For example, the amount of sulfur (S) may increase as it leaves the core.

For example, the quantum dot body with the core-shell structure may include: a core, where the core includes a third semiconductor compound, and the third semiconductor compound includes indium (In), as well as at least one of zinc (Zn) and phosphorus (P); and a shell provided on at least a portion of the core and including a fourth semiconductor compound with a composition different from that of the core.

In the third semiconductor compound based on In-Zn-P, the molar ratio of zinc (Zn) to indium (In) can be greater than or equal to about 25. For example, in the third semiconductor compound based on In-Zn-P, the molar ratio of zinc (Zn) to indium (In) can be greater than or equal to about 28, greater than or equal to about 29, or greater than or equal to about 30. For example, in the third semiconductor compound based on In-Zn-P, the molar ratio of zinc (Zn) to indium (In) may be less than or equal to about 55, such as less than or equal to about 50, less than or equal to about 45, less than or equal to about 40, less than or equal to about 35, less than or equal to about 34, less than or equal to about 33, or less than or equal to about 32.

The fourth semiconductor compound may include, for example, II-VI semiconductor compound, III-V semiconductor compound, IV-VI semiconductor compound, IV semiconductor, I-III-VI semiconductor compound, I-II-IV-VI semiconductor compound, II-III-V semiconductor compound, or combinations thereof. The examples of II-VI semiconductor compound, III-V semiconductor compound, IV-VI semiconductor compound, IV semiconductor, I-III-VI semiconductor compound, I-II-IV-VI semiconductor compound, and II-III-V semiconductor compound are the same as described above.

For example, the fourth semiconductor compound may include zinc (Zn) and sulfur (S), as well as optional selenium (Se). For example, the shell may include ZnSeS, ZnSe, ZnS, or a combination thereof. For example, the shell may include at least one inner shell provided near the core and the outermost shell provided at the outermost side of the quantum dot body. At least one of the inner shell and the outermost shell may include a fourth semiconductor compound ZnS, ZnSe, or ZnSeS.

In the present disclosure, the quantum dot ligand can form coordination bond with the surface of the quantum dot body through the coordination group, thereby connecting the quantum dot ligand to the surface of the quantum dot body.

The dissolving group is selected from a polar group. The polar group refers to a group of which the positive and negative charge centers do not overlap. The polarity of such group can be characterized by a dipole moment. The dipole moment is the product of the distance 1 between positive and negative charge centers and the amount of charge ± q carried at the charge center. It is a vector with a direction specified as pointing from the positive center to the negative center, denoted by the symbol µ, µ= ql, the dipole moment of the polar group µ> 0.5. That is, the dipole moment of the dissolving group µ> 0.5. The polar group exhibits affinity for polar solvents such as propylene glycol methyl ether acetate (PGMEA) and determines the hydrophilic property of the quantum dot ligand of the present disclosure.

Furthermore, a strong polar group can be selected for the dissolving group, i.e., group of µ> 0.7.

The linking group is selected from an alkylene group. The alkylene group can be either a straight chain alkylene group or a branched chain alkylene group.

In this disclosure, the photosensitive group can break bond under light condition, which means that when exposed to light such as ultraviolet light, a chemical bond in the photosensitive group can directly break, thereby decomposing the quantum dot ligand from the breaking position of the photosensitive group. Specifically, the quantum dot ligand is decomposed into a first unit including the coordination group and the linking group, as well as a second unit including the dissolving group.

It should be noted that the first unit formed by decomposition also includes some structures in the photosensitive group, and similarly, the second unit also includes some structures in the photosensitive group, depending on the bond breaking position of the photosensitive group.

The polarity of the first unit formed by decomposition is smaller than that of the second unit. For example, the first unit has a weak polarity, while the second unit has a polarity or a strong polarity. For example, the dielectric constant of the first unit is smaller than that of the second unit. Or, for the first unit, µ≤ 0.5, for the second unit, µ>0.5, or for the second unit, µ>0.7.

In this disclosure, the first unit has a weak polarity and has no affinity for polar solvents, while the second unit has a strong polarity and has affinity for polar solvents, providing a basis for the quantum dot film layer to be formed by a photolithographic process.

In some embodiments of the present disclosure, after the photosensitive group breaks the bond under light condition, the end of the first unit forms an amino (-NH2), hydroxyl (-OH), or carboxyl (-COOH) group.

In some embodiments of the present disclosure, Y further includes a solubilizing group. The solubilizing group is connected between the photosensitive group and the dissolving group, and the solubilizing group is selected from a structure including 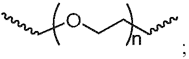 where n is any integer selected from 1 to 9. The solubilizing group can be connected to other groups through 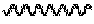 .

In this embodiment, the solubilizing group helps to enhance the solubility of the quantum dot ligand. The solubilizing group includes 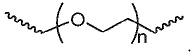 This structure has good water solubility and good compatibility with polar solvents.

In this disclosure, n is selected from any integer between 1 and 9, which can be selected from 1, 2, 3, 4, 5, 6, 7, 8, or 9.

When Y also includes the solubilizing group, the second unit also includes the solubilizing group.

In some embodiments of the present disclosure, the coordination group is selected from amino, carboxylic acid, sulfhydryl, phosphine or phosphine oxide groups. For example, when the coordination group is selected from the sulfhydryl group, the S atom in the sulfhydryl group forms a coordination bond with the surface of ZnSe/CdSe quantum dot. When the coordination group is selected from the amino group, the N atom in the amino group forms a coordination bond with the surface of ZnSe/CdSe quantum dot.

In some embodiments of the present disclosure, the linking group is selected from an alkylene group with a carbon atom number of 2 to 8. The linking group within this range has good flexibility of chain segments and low resistance, which facilitates the binding of the quantum dot ligand to the surface of the quantum dot. Moreover, the linking group within this range does not affect the injection of charge carriers (electrons and holes) into the quantum dot, thus not affecting the performance of the quantum dot light-emitting device.

Specifically, the carbon atom number of the alkylene group can be 2, 3, 4, 5, 6, 7, or 8. The alkylene group can be either a straight chain alkylene group or a branched chain alkylene group, such as ethylidene, n-propylidene, isopropylidene, n-butylidene, isobutylidene, sec-butylidene, tertbutylidene, pentylidene, hexylidene, heptanylidene, octylidene, etc.

In some embodiments of the present disclosure, the dissolving group is selected from the quaternary ammonium salt group. The quaternary ammonium salt group is a strong polar group, which helps to enhance the solubility of the quantum dot ligand in polar solvents such as propylene glycol methyl ether acetate (PGMEA).

Further, the dissolving group is selected from the structure of

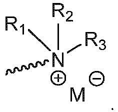

R₁, R₂, and R₃ are same or different, which are independently selected from methyl group and ethyl group; M is selected from halogen atoms.

The halogen atoms can be selected from fluorine, chlorine, bromine, and iodine, and preferably, chlorine atom and bromine atom.

In this disclosure, the description "independently selected from ..." is broadly construed to mean either that the specific options expressed between the same symbols in different groups do not affect each other, or that the specific options expressed between the same symbols in the same groups do not affect each other.

In some embodiments of the present disclousre, not falling within the scope of the claims, the photosensitive group is selected from the structure including

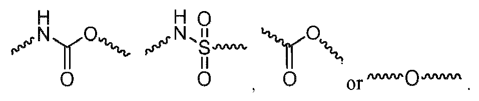

According to the invention, the photosensitive group is selected from a group including the following structures:

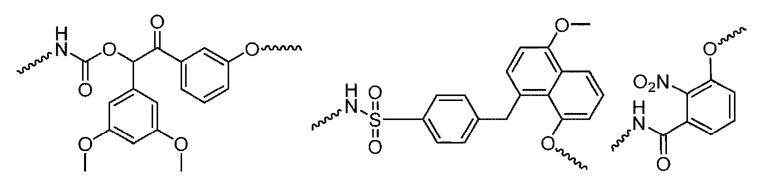

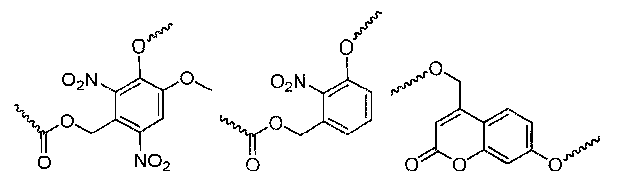

In some embodiments of the present disclosure, the quantum dot ligand is selected from a group including the following structures: 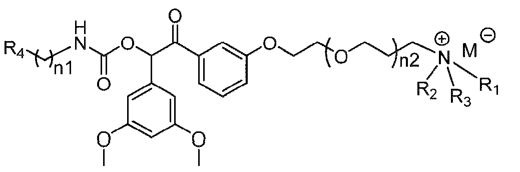 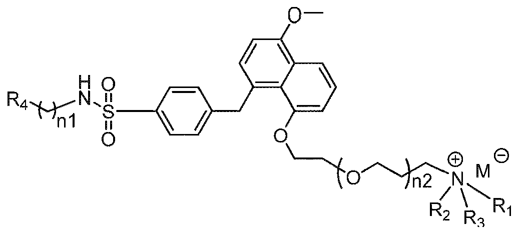 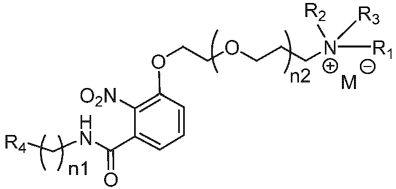 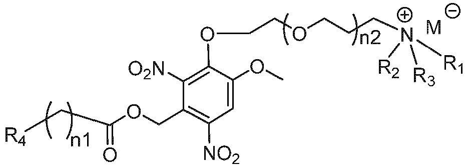 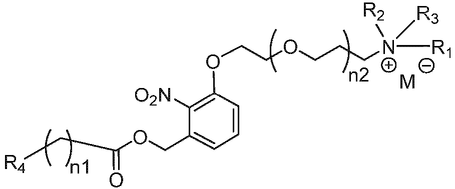 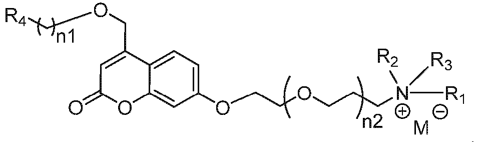
R₁, R₂, and R₃ are same or different, and are independently selected from methyl group and ethyl group;
M is selected from chlorine or bromine;
R₄ is selected from amino, carboxylic acid, sulfhydryl, phosphine or phosphine oxide groups;
nl is selected from any integer from 2 to 8;
n2 is selected from any integer from 1 to 9.

It is to be noted herein that the positions of the quantum dot ligands of the present disclosure where bond breakings occur under light condition are shown at the following dashed positions, wherein the left side of the dashed line is the first unit and the right side of the dashed line is the second unit:

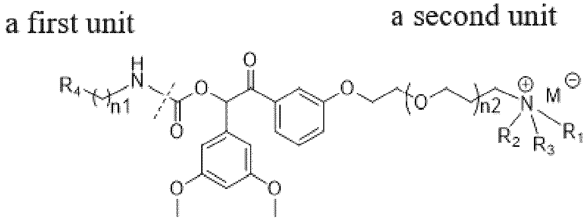

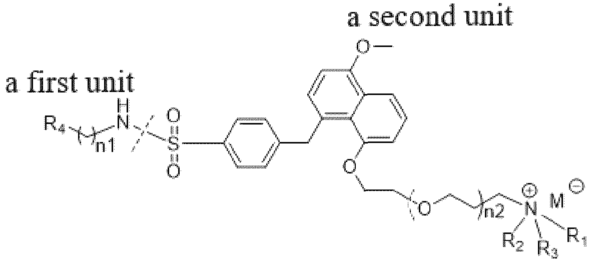

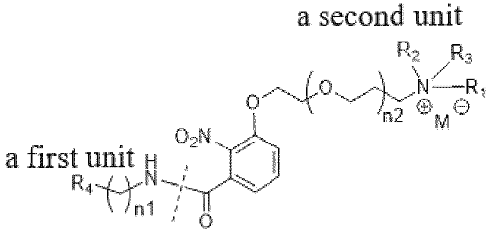

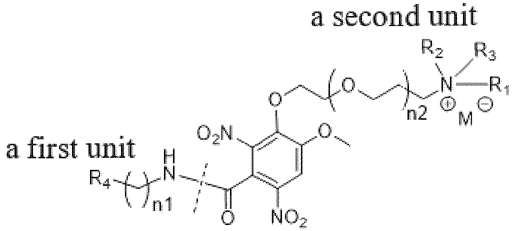

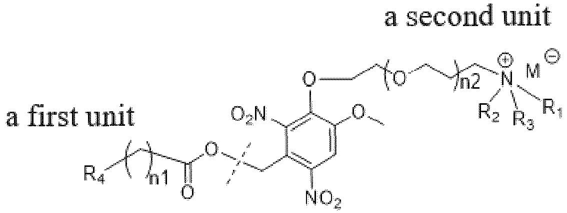

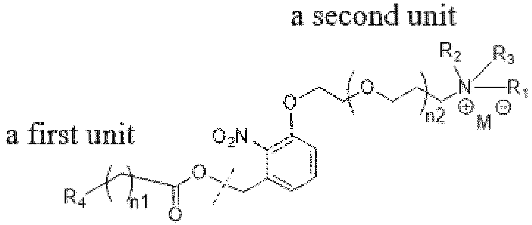

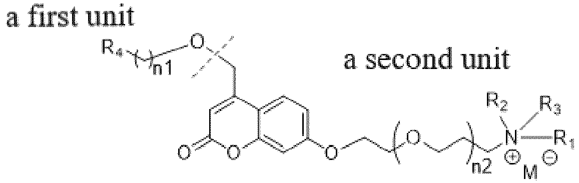

In some embodiments of the present disclosure, the quantum dot ligand is selected from a group including the following structures: 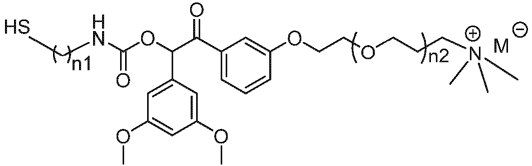 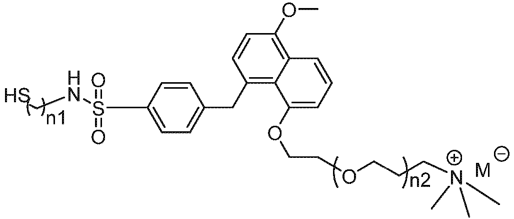 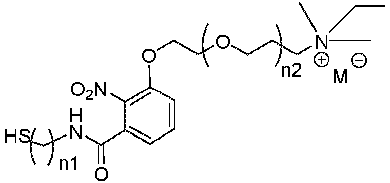 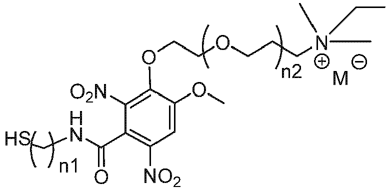 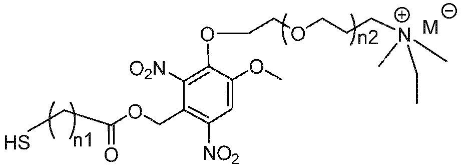 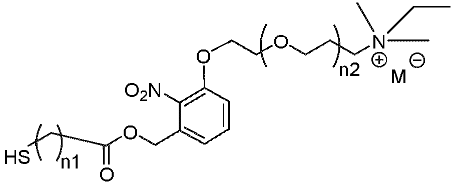 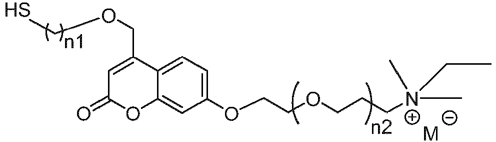
M is selected from chlorine or bromine.
n1 is selected from any integer from 2 to 8.
n2 is selected from any integer from 1 to 9.

The present disclosure also provides a quantum dot-ligand material including a quantum dot body and a quantum dot ligand in any of the above embodiments, where the quantum dot ligand is connected to the quantum dot body via a coordination bond.

The quantum dot-ligand material provided in the present disclosure is used for the material with a dissolving group attached thereto, and thus, can be soluble in polar solvents such as propylene glycol methyl ether acetate (PGMEA) and alcohols. When this quantum dot-ligand material is exposed to light, the photosensitive group contained therein breaks bond, thereby decomposing the quantum dot-ligand material into a first material including a quantum dot, a coordination group, and a linking group, as well as a second material including a dissolving group. The first material has a weak polarity and is insoluble in polar solvents such as propylene glycol methyl ether acetate, and the second material has a strong polarity and is soluble in propylene glycol methyl ether acetate.

In addition, the propylene glycol methyl ether acetate polar solvent, which is a commonly used solvent in the current display device preparation process, is an environmentally friendly solvent with multi-functional groups. The quantum dot-ligand material provided by the present disclosure can be applied to existing production lines for display devices, and can be produced in large quantities without increasing generation costs.

The present disclosure also provides a method of preparing a quantum dot film layer, including:
Step S100, providing the quantum dot-ligand material described above;
Step S200, coating the quantum dot-ligand solution on a substrate, and performing an exposure treatment such that the photosensitive group in the quantum dot-ligand material undergoes bond breaking to decompose the quantum dot-ligand material into a first material including the quantum dot, the coordination group, and the linking group, as well as a second material including the dissolving group;
Step S300, performing a development process, where the second material is soluble in the development solution, the first material is insoluble in the development solution, and the first material forms the quantum dot film layer.

In some embodiments of the present disclosure, the end of the first material is selected from one or more of an amino group, a cyano group, or a carboxyl group.

When the quantum dot ligand further includes a solubilizing group, the second material further includes the solubilizing group.

In the present disclosure, the structure of the second material is the same as the structure of the second unit.

The present disclosure also provides a quantum dot light-emitting device including a functional layer. The functional layer includes a quantum dot film layer, and the quantum dot film layer includes the above-described first unit.

In some embodiments of the present disclosure, the quantum dot film layer includes a plurality of sub-units. The sub-units include a first color quantum dot and a second color quantum dot. The material of the first color quantum dot includes a first color quantum dot body and the first unit described above, i.e., the material of the first color quantum dot is a material formed by the photosensitive group after the bond breaking occurs normally under light condition. The material of the second color quantum dot includes a second color quantum dot body and a quantum dot ligand as described above in which no bond breaking occurs, i.e., the second color quantum dot is a material which remains on the first color quantum dot without the photosensitive group breaking bond. In at least one sub-unit of the plurality of sub-units, the mass ratio of the material of the second color quantum dot to the material of the first color quantum dot is less than 10⁻³. Specifically, it may be, but is not limited to, 10⁻³, 2.5×10⁻⁴, 10⁻⁴, 5×10⁻⁵, 2.5 × 10⁻⁵, 10⁻⁶, 10⁻⁷, 10⁻⁸ or infinitely approaching 0.

In some specific embodiments, a certain semiconductor contained in the quantum dot body can be used to characterize the mass of the material of the first color quantum dot and the material of the second color quantum dot. For example, the present disclosure utilizes quantum dot bodies containing cadmium to fabricate quantum dot light-emitting devices. The cadmium content in the material of the second color quantum dot can be 0.4-1 µg/cm², particularly can be 0.45 µg/cm², 0.5 µg/cm², 0.55 µg/cm², 0.6 µg/cm², 0.7 µg/cm², 0.75 µg/cm², 0.8 µg/cm², 0.85 µg/cm², 0.9 µg/cm², 0.95 µg/cm², 1 µg/cm², but not limited to this. The cadmium content in the material of the second color quantum dot is less than 10⁻⁴ µg/cm². For example, it can be 10⁻⁴ µg/cm², 10⁻⁵ µg/cm², 10⁻⁶ µg/cm², 10⁻⁷ µg/cm², 10⁻⁸ µg/cm² or infinitely approaching 0, but not limited to this.

In addition, the present disclosure can also use quantum dot bodies not containing cadmium to fabricate quantum dot light-emitting devices. For example, it uses quantum dot bodies containing indium. At this point, the indium content in the material of the second color quantum dot can be 0.4-1 µg/cm², particularly can be 0.45 µg/cm², 0.5 µg/cm², 0.55 µg/cm², 0.6 µg/cm², 0.7 µg/cm², 0.75 µg/cm², 0.8 µg/cm², 0.85 µg/cm², 0.9 µg/cm², 0.95 µg/cm², 1 µg/cm², but not limited to this. The indium content in the material of the second color quantum dot is less than 10⁻⁴ µg/cm². For example, it can be 10⁻⁴ µg/cm², 10⁻⁵ µg/cm², 10⁻⁶ µg/cm², 10⁻⁷ µg/cm², 10⁻⁸ µg/cm² or infinitely approaching 0, but not limited to this.

The quantum dot light-emitting devices provided in the present disclosure can be electroluminescent quantum dot light-emitting devices or photoluminescent quantum dot light-emitting devices.

In some embodiments of the present disclosure, the quantum dot light-emitting device further includes an anode and a cathode, with a functional layer provided between the anode and the cathode. The functional layer also includes a hole injection layer, a hole transport layer, an electron transport layer, and an electron injection layer.

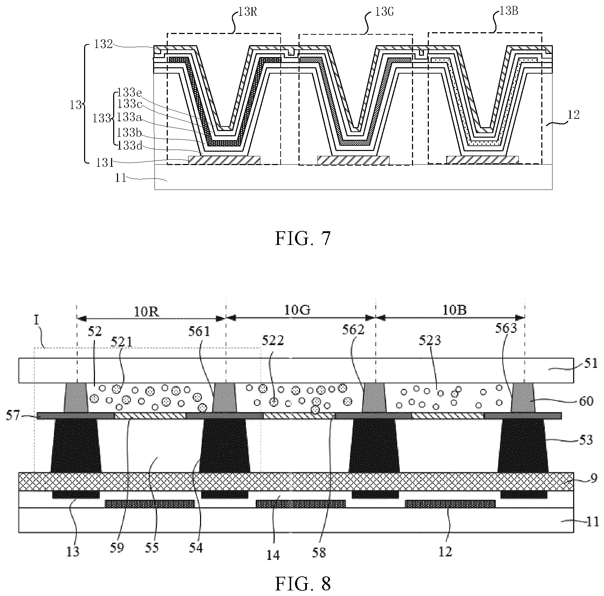

As shown in Fig. 7, in a specific embodiment of the present disclosure, the quantum dot light-emitting device may include a substrate 11 as well as a first electrode 131, a hole injection layer 133d, a hole transport layer 133b, a quantum dot film layer 133a, an electron transport layer 133c, an electron injection layer 133e, and a second electrode 132. The first electrode 131, the hole injection layer 133d, the hole transport layer 133b, the quantum dot film layer 133a, the electron transport layer 133c, the electron injection layer 133e, and the second electrode 132 are sequentially stacked on one side of the substrate 11.

The substrate 11 can be a component providing a base surface in which the display device layer DP-OEL is set. The substrate 11 can be: inorganic materials such as glass substrate or metal substrate; organic materials such as polycarbonate, polymethyl methacrylate, polyethylene terephthalate, polyethylene naphthalate, polyamide, polyethersulfone, or combinations thereof; silicon wafers; or composite material layers, etc.

One of the first electrode 131 and the second electrode 132 is an anode and the other is a cathode. For example, the first electrode 131 may be an anode and the second electrode 132 may be a cathode. For example, the first electrode 131 may be a cathode and the second electrode 132 may be an anode.

The anode may include a conductor having a high work function such as a metal, a conductive metal oxide, or a combination thereof. The anode may include, for example, a metal that may be nickel, platinum, vanadium, chromium, copper, zinc, or gold. or an alloy thereof; a conductive metal oxide that may be zinc oxide, indium oxide, tin oxide, indium-tin oxide (ITO), indium-zinc oxide (IZO), or fluorine-doped tin oxide; or a combination of the metal and the conductive metal oxide that may be, but is not limited to, ZnO and Al, or SnO2 and Sb.

The cathode may include a conductor having a lower work function than the anode, such as a metal, a conductive metal oxide, and/or a conductive polymer. The cathode may include, e.g., a metal that may be aluminum, magnesium, calcium, sodium, potassium, titanium, indium, yttrium, lithium, gadolinium, silver, tin, lead, cesium, barium, and the like, or alloys thereof; a multilayer structure e.g., LiF/Al, Li₂O/Al, Liq/Al, LiF/Ca, and BaF₂/Ca, but is not limited to this.

The work function of the anode may be higher than the work function of the cathode, e.g., the work function of the anode may be, for example, from about 4.5 eV to about 5.0 eV and the work function of the cathode may be from about 4.0 eV to about 4.7 eV. Within this range, the work function of the anode may be, for example, from about 4.6 eV to about 4.9 eV or from about 4.6eV to about 4.8 eV and the work function of the cathode may be, for example, from about 4.0 eV to about 4.6 eV or from about 4.3 eV to about 4.6 eV.

The first electrode 131 and the second electrode 132 may be a transmissive electrode, a partially transmissive partially reflective electrode, or a reflective electrode. The transmissive electrode or the partially transmissive partially reflective electrode may include: an electrically conductive oxide such as zinc oxide, indium oxide, tin oxide, indium-tin oxide (ITO), indium-zinc-oxide (IZO), or fluorine-doped tin oxide, or a thin metal layer. The reflective electrode may include: a reflective metal, e.g., an opaque conductor such as aluminum (Al), silver (Ag), or gold (Au), and the first electrode and the second electrode may be of a single-layer or multi-layer structure.

At least one of the first electrode 131 or the second electrode 132 may be connected to an auxiliary electrode. If connected to the auxiliary electrode, the resistance of the second electrode 132 may be reduced.

The hole transfer layer 133b and the hole injection layer 133d are provided between the first electrode 131 and the quantum dot film layer 133a. The hole transfer layer 133b is provided between the first electrode 131 and the quantum dot film layer 133a near the quantum dot film layer 133a, and the hole injection layer 133d is provided between the first electrode 131 and the quantum dot film layer 133a near the first electrode 131. The hole injection layer 133d may facilitate the injection of holes from the first electrode, and the hole transport layer 133b may efficiently transfer the injected holes to the quantum dot film layer 133a. The hole transport layer 133b and the hole injection layer 144d may have one or two or more layers, respectively, and in a broader sense may include an electron blocking layer.

The hole transport layer 133b and the hole injection layer 133d may each have a HOMO energy level between the work function of the first electrode 131 and the HOMO energy level of the quantum dot film layer 133a. For example, the work function of the first electrode 131, the HOMO energy level of the hole-injection layer 133d, the HOMO energy level of the hole-transport layer 133b, and the HOMO energy level of the quantum dot film layer 133a may be progressively deeper and may be, for example, stepped.

The hole transport layer 133b may have a relatively deep HOMO energy level to match the HOMO energy level of the quantum dot film layer 133a. As a result, the mobility of holes transferred from the hole transport layer 133b to the quantum dot film layer may be improved.

The HOMO energy level of the hole transport layer 133b may be equal to the HOMO energy level of the quantum dot film layer 133a or less than the HOMO energy level of the quantum dot film layer 133a within a range of about 1.0 eV or less. For example, the difference between the HOMO energy levels of the hole transport layer 133b and the quantum dot film layer 133a may be from about 0 eV to about 1.0 eV, in a range of, for example, from about 0.01 eV to about 0.8 eV, in a range of, for example, from about 0.01 eV to about 0.7 eV, in a range of, for example, from about 0.01 eV to about 0.5 eV, in a range of, for example, from about 0.01 eV to about 0.4eV, for example about 0.01eV to about 0.3eV, for example about 0.01eV to about 0.2eV, for example about 0.01eV to about 0.1eV.

The HOMO energy level of the hole transport layer 133b may be, for example, greater than or equal to about 5.0 eV, in the range of, for example, greater than or equal to about 5.2 eV, in the range of, for example, greater than or equal to about 5.4 eV, in the range of, for example, greater than or equal to about 5.6 eV, in the range of, for example, greater than or equal to about 5.8 eV.

For example, the HOMO energy level of the hole transport layer 133b may be from about 5.0 eV to about 7.0 eV, in the range above, such as from about 5.2 eV to about 6.8 eV, in the range above, such as from about 5.4 eV to about 6.8 eV, for example from about 5.4eV to about 6.7 eV, for example from about 5.4eV to about 6.5 eV, for example from about 5.4eV to about 6.3 eV, e.g., from about 5.4eV to about 6.2eV, e.g., from about 5.4eV to about 6.1eV, e.g., from about 5.6eV to about 7.0eV, e.g., from about 5.6eV to about 6.8eV, e.g., from about 5.6eV to about 6.7eV, e.g., from about 5.6eV to about 6.5eV, e.g., from about 5.6eV to about 6.3eV, e.g., from about 5.6eV to about 6.2eV, e.g., from about 5.6eV to about 6.1eV, e.g., from about 5.8eV to about 7.0eV, e.g., from about 5.8eV to about 6.8eV, e.g., from about 5.8eV to about 6.7eV, e.g., from about 5.8eV to about 6.5eV, e.g., from about 5.8eV to about 6.3eV, e.g., from about 5.8eV to about 6.2eV, e.g., from about 5.8eV to about 6.1eV.

The hole transport layer 133b and the hole injection layer 133d may include materials that satisfy the energy levels without particular limitation, and may include, for example, at least one selected from the following: poly(9,9-dioctyl-fluorene-co-N-(4-butylphenyl)-diphenylamine) (TFB), poly(N,N'-bis-4-butylphenyl-N,N'-biphenyl) benzidine (poly TPD), polyarylamine (polyarylamine), poly(N-vinylcarbazole), poly(3,4-ethylenedioxythiophene) (PEDOT), poly(3,4-ethylenedioxythiophene):polystyrene sulfonate (PEDOT:PSS), polyaniline, polypyrrole, N,N,N',N'-tetrakis(4-methoxyphenyl)-benzidine (TPD), 4,4'-bis[N-(1-naphthalenyl)-N-phenyl-amino]biphenyl (α-NPD), m-MTDATA (4,4',4"-tris[phenyl(m-tolyl)amino]triphenylamine), 4,4',4"-tris(N-carbazolyl)-triphenylamine (TCTA), 1,1-bis[(di-4-tolylamino)phenyl]cyclohexane (TAPC), p-type metal oxides (e.g., NiO, WO3, MoO3, etc.), carbon-based materials such as graphene oxides, phthalocyanine compounds (e.g., copper phthalocyanine); N,N'-diphenyl-N,N'-bis-[4-(phenyl-m-tolyl-amino)-phenyl]-biphenyl-4,4'-diamine (DNTPD), 4,4',4"-tris(3-methylphenylphenylamino)triphenylamine (m-MTDATA), 4,4',4"-tris(N,N-diphenylamino)triphenylamine (TDATA), 4.,4' 4"-tris{N-(2-naphthyl)-N-phenylamino}-triphenylamine (2-TNATA), poly(3,4-ethylenedioxythiophene)/poly(4-styrenesulfonate) (PEDOT/PSS), polyaniline/dodecylbenzenesulfonic acid (PANI/DBSA), polyaniline/camphorsulfonic acid (PANI/CSA), polyaniline/poly(4-styrenesulfonate) (PANI/PSS), N,N'-bis(naphthalen-1-yl)-N,N'-diphenylbiphenyl-benzidine (NPB), triphenylamine-containing polyetherketone (TPAPEK), 4-isopropyl-4'-methyldiphenyliodonium tetrakis(pentafluorophenyl)borate and/or dipyrazino[2,3-f:2',3'-h]quinoxaline-2,3,6,7,10,11-hexamethylnitrile (HAT-CN), carbazole derivatives (e.g., N-phenylcarbazole and/or polyvinylcarbazole), fluorine derivatives, N,N'-bis(3-methylphenyl)-N,N'-diphenyl-[1,1-biphenylyl]-4,4'-diamine (TPD), triphenylamine derivatives (e.g., 4,4',4"-tris(N-carbazolyl)triphenylamine (TCTA)), N,N'-bis(naphthalen-1-yl)-N,N'-diphenyl-benzidine (NPB), 4,4'-cyclohexylidenebis[N,N-bis(4-methylphenyl)aniline] (TAPC), 4,4'-bis[N,N'-(3-methylphenyl)amino]-3,3'-dimethylbiphenyl (HMTPD), 1,3-bis(N-carbazolyl)benzene (mCP), and combinations thereof, but not limited thereto.

One or both of the hole transport layer and the hole injection layer may be omitted.

The hole transport layer 133b and the hole injection layer 133d may be formed using one or more suitable methods (e.g., vacuum deposition, spin coating, casting, Langmuir-Blodgett (LB), sputtering, inkjet printing, laser printing, and/or laser-induced thermal imaging (LITI)).

Quantum dots of different sizes in the quantum dot film layer may correspond to emitting different colors of light and forming sub-pixels of different colors, such as a first color sub-pixel 13G, a second color sub-pixel 13B, and a third color sub-pixel 12R, respectively.

The electron transmission layer 133c and the electron injection layer 133e are provided between the second electrode 132 and the quantum dot film layer 133a. The electron transmission layer 133c is provided between the second electrode 132 and the quantum dot film layer 133a near the quantum dot film layer 133a, and the electron injection layer 133e is provided between the second electrode 132 and the quantum dot film layer 133a near the second electrode 132. The electron injection layer 133e may facilitate the injection of electrons from the second electrode, and the electron transport layer 133c may efficiently transfer the injected electrons to the quantum dot film layer 133a. The electron transport layer 133c and the electron injection layer 133e may have one or two or more layers, respectively, and may include a hole blocking layer in a broad sense.

For example, the electron injection layer 133e may be in contact with the second electrode 132.

For example, the electron transport layer 133c may be in contact with the quantum dot film layer 133a.

For example, the electron transport layer 133c and the electron injection layer 133e may be in contact with each other. One or both of the electron transport layer and the electron injection layer may be omitted.

For example, the LUMO energy levels of the second electrode 132, the electron injection layer 133e, the electron transport layer 133c, and the quantum dot film layer 133a may become progressively shallower. For example, the LUMO energy level of the electron injection layer 133e may be shallower than the work function of the second electrode 132, and the LUMO energy level of the electron transport layer 133c may be shallower than the LUMO energy level of the electron injection layer 133e, and the LUMO energy level of the quantum dot film layer 133a may be shallower than the LUMO energy level of the electron transport layer 133c. That is, the work function of the second electrode 132, the LUMO energy level of the electron injection layer 133e, the LUMO energy level of the electron transport layer 133c, and the LUMO energy level of the quantum dot film layer 133a may have an order (cascade) energy level that decreases gradually in one direction.

The electron transport layer 133c may include a first inorganic nanoparticle. The first inorganic nanoparticle may be, for example, an oxide nanoparticle, and may be, for example, a metal oxide nanoparticle.

The first inorganic nanoparticle may be a two-dimensional or three-dimensional nanoparticle having an average particle diameter of: less than or equal to about 10 nm, in the range of less than or equal to about 8 nm, less than or equal to about 7 nm, less than or equal to about 5 nm, less than or equal to about 4 nm, or less than or equal to about 3.5 nm, or in the range of from about 1 nm to about 10 nm, from about 1 nm to about 9 nm, from about 1 nm to about 8 nm, from about 1 nm to about 7 nm, from about 1 nm to about 5 nm, from about 1 nm to about 4 nm, or from about 1 nm to about 3.5 nm.

For example, the first inorganic nanoparticle may be a metal oxide nanoparticle. The metal oxide nanoparticle includes at least one of: zinc (Zn), magnesium (Mg), cobalt (Co), nickel (Ni), gallium (Ga), aluminum (Al), calcium (Ca), zirconium (Zr), tungsten (W), lithium (Li), titanium (Ti), tantalum (Ta), tin (Sn), hafnium (Hf), and barium (Ba).

As an example, the first inorganic nanoparticle may include a metal oxide nanoparticle containing zinc (Zn) and may include a metal oxide nanoparticle denoted by Zn1-xQxO (0≤x<0.5). Herein, Q is at least one metal different from Zn, such as magnesium (Mg), cobalt (Co), nickel (Ni), gallium (Ga), aluminum (Al), calcium (Ca), zirconium (Zr), tungsten (W), lithium (Li), titanium (Ti), tantalum (Ta), tin (Sn), hafnium (Hf), silicon (Si), barium (Ba), or a combination thereof.

For example, Q may include magnesium (Mg).

For example, x may be in the range of 0.01 ≤ x ≤ 0.3, e.g., 0.01 ≤ x ≤ 0.2.

The LUMO energy level of the electron transport layer 16 may be a value between the LUMO energy level of the quantum dot film layer 133a and the LUMO energy level of the electron injection layer 17, and may be from about 3.2 eV to about 4.8 eV, from about 3.2 eV to about 4.6 eV, from about 3.2 eV to about 4.5 eV, from about 3.2 eV to about 4.3 eV, from about 3.2 eV to about 4.1 eV, from about 3.4eV to about 4.1eV, from about 3.5eV to about 4.6eV, from about 3.6eV to about 4.6eV, from about 3.6eV to about 4.3eV, from about 3.6eV to about 4.1eV, about 3.6eV to about 3.9eV, from about 3.7eV to about 4.6eV, from about 3.7eV to about 4.3eV, from about 3.7eV to about 4.1eV, or from about 3.7 eV to about 3.9 eV.

The thickness of the electron transport layer 133c may be greater than about 10 nm and less than or equal to about 80 nm, and in a range of greater than about 10 nm and less than or equal to about 70 nm, greater than about 10 nm and less than or equal to about 60 nm, greater than about 10 nm and less than or equal to about 50 nm, greater than about 10 nm and less than or equal to about 40 nm, or greater than about 10 nm and less than or equal to about 30 nm.

The LUMO energy level of the electron injection layer 133e may be between the work function of the second electrode 132 and the LUMO energy level of the electron transport layer. For example, the difference between the work function of the second electrode 132 and the LUMO energy level of the electron injection layer 133e may be less than about 0.5 eV, from about 0.001 eV to about 0.5 eV, from about 0.001 eV to about 0.4 eV, or from about 0.001 eV to about 0.3 eV. By way of example, the difference between the LUMO energy level of the electron injection layer 133e and the LUMO energy level of the electron transport layer may be less than about 0.5 eV, from about 0.001 eV to about 0.5 eV, from about 0.001 eV to about 0.4 eV, or from about 0.001 eV to about 0.3 eV. As a result, the electrons may be readily injected from the second electrode 132 into the electron injection layer 133e to reduce the drive voltage of the quantum dot device, and the electrons may be efficiently transferred from the electron injection layer 133e to the electron transport layer to increase efficiency. Within a range satisfying the foregoing energy level, the LUMO energy level of the electron injection layer may be from about 3.4 eV to about 4.8 eV, from about 3.4 eV to about 4.6 eV, from about 3.4 eV to about 4.5 eV, from about 3.6 eV to about 4.8 eV, from about 3.6 eV to about 4.6 eV, from about 3.6 eV to about 4.5 eV, from about 3.6 eV to about 4.3 eV, from about 3.9 eV to about 4.8eV, from about 3.9eV to about 4.6eV, from about 3.9eV to about 4.5eV, or from about 3.9eV to about 4.3eV.

The electron injection layer 133e can be thinner than the electron transport layer 133c. For example, the thickness of the electron injection layer 133e may be about 0.01 to about 0.8 times, about 0.01 to about 0.7 times, about 0.01 to about 0.5 times, about 0.1 to about 0.8 times, about 0.1 to about 0.7 times, or about 0.1 to about 0.5 times the thickness of the electron transport layer 133c. The thickness of the electron injection layer 17 may be, for example, less than or equal to about 10nm, less than or equal to about 7nm, or less than or equal to about 5nm. Within the range, the thickness of the electron injection layer 17 can be from about 1nm to about 10nm, from about 1nm to about 8nm, from about 1nm to about 7nm, or from about 1nm to about 5nm.

One or more suitable methods (such as vacuum deposition, spin coating, casting, Langmuir Blodget (LB) method, inkjet printing, sputtering, laser printing, and/or laser-induced thermal imaging (LITI)) can be used to form the electron transport layer 133c and the electron injection layer 133e.

In some embodiments of the present disclosure, the quantum dot light-emitting device can also be a photoluminescence quantum dot device containing a light-emitting unit, with the quantum dot film layer located on one side of the light-emitting unit.

As shown in Fig. 8, the quantum dot light-emitting device according to the embodiment of the present disclosure may include a first substrate and a second substrate. The first substrate and the second substrate may be disposed opposite to each other. For example, the first substrate can be a substrate provided with a light source and other components, and the second substrate can be a substrate provided with a color film and other components.

The first substrate may include a first substrate 11 and a plurality of light-emitting units 12 provided on the first substrate 11.

The second substrate may include: a second substrate 51; a quantum dot film layer provided on the second substrate 51, where the quantum dot film layer at least includes a plurality of quantum dot structures; a plurality of extinction structures 53 provided on one side of the quantum dot film layer facing the first substrate, where a first channel 54 is formed between any two adjacent extinction structures 53; and a plurality of first optical structures 55 provided on one side of the quantum dot film layer facing the first substrate, where a plurality of first optical structures 55 are located respectively in the first channels 54 between any two adjacent extinction structures 53.

The quantum dot light-emitting device may also include a filling material portion 9 arranged between the first substrate and the second substrate.

In the embodiment of the present disclosure, the refractive index of the material in the filling material portion 9 is greater than that of the material in the first optical structure 55, and the extinction structure 53 includes a light-absorbing material.

As shown in Fig. 8, the orthographic projection of a plurality of light-emitting units 12 on the first substrate 11 overlaps at least partially with the orthographic projection of a plurality of first optical structures 55 on the first substrate 11; the orthographic projection of the quantum dot film layer on the first substrate 11 overlaps at least partially with the orthographic projection of a plurality of first optical structures 55 on the first substrate 11; and the orthographic projection of a plurality of first optical structures 55 on the first substrate 11 falls into the orthographic projection of the filling material portion 9 on the first substrate 11. The first substrate 11 and the second substrate 51 may be rigid or flexible substrates, including but not limited to glass substrates or polyimide (PI) substrates.

In embodiments of the present disclosure, the plurality of light emitting units 12 may include a plurality of organic light emitting diodes or a plurality of inorganic light emitting diodes, such as Mini LEDs or Micro LEDs.

In embodiments of the present disclosure, the quantum dot light emitting device may include a plurality of sub-pixels I, such as an area surrounded by a dashed box. The sub-pixels I may be a third color sub-pixel 10R for emitting light having a first wavelength range, a first color sub-pixel 10G for emitting light having a second wavelength range, and a second color sub-pixel 10B for emitting light having a third wavelength range. each of the sub-pixels may include a sub-pixel opening. For example, the third color sub-pixel 10R may include a first sub-pixel opening 561, the first color sub-pixel 10G may include a second sub-pixel opening 562, and the second color sub-pixel 10B may include a third sub-pixel opening 563. The first color, the second color, and the third color may refer to green, blue, and red, respectively. Of course, the quantum dot light-emitting device may also include pixels for emitting other colors, such as pixels for emitting yellow light, and embodiments of the present disclosure do not specifically limit them.

The quantum dot film layer may include a plurality of quantum dot structures for emitting different colors. The quantum dot structure includes the first unit of the present disclosure. For example, the quantum dot structure includes a quantum dot body and a first unit, the first unit being bonded to the surface of the quantum dot body. For example, the third color sub-pixel 10R may include a first quantum dot structure 521 for emitting light having a first wavelength range, and the first color sub-pixel 10G may include a second quantum dot structure 522 for emitting light having a second wavelength range. Of course, the quantum dot film layer may also include a quantum dot structure for emitting light having other wavelength range, such as a quantum dot structure for emitting yellow light.

The second substrate may also include a plurality of light-blocking structures 57 provided on the second substrate 51. The plurality of light-blocking structures 57 are disposed between the layer in which the extinction structure 53 is disposed and the layer in which the quantum dot film layer 52 is disposed. For example, the light-blocking structure 57 includes a light-blocking material.

A second channel 58 is formed between any two adjacent light-blocking structures 57. The orthographic projection of the second channel 58 on the second substrate 51 falls within the orthographic projection of the first channel 54 on the second substrate 51. The plurality of first channels 54 and the plurality of second channels 58 are connected respectively to form a plurality of light-entry channels.

The second substrate may further include a plurality of quantum dot protection structures 59 provided on the second substrate 51. The plurality of quantum dot protection structures 59 are disposed between the quantum dot film layer 52 and the first optical structure 55. The orthographic projections of the plurality of quantum dot protection structures 59 on the second substrate 51 are located within the orthographic projections of the plurality of second channels 58 on the second substrate 51, respectively. In this manner, the plurality of quantum dot protection structures 59 protect the quantum dot structures disposed in the respective pixel openings, respectively.

The second substrate may further include a plurality of retaining wall structures 60 provided on the second substrate 51. The plurality of retaining wall structures 60 are disposed between the second substrate 51 and the plurality of extinction structures 53. The orthographic projections of the plurality of retaining wall structures 60 on the second substrate 51 are disposed within the orthographic projections of the plurality of extinction structures 53 on the second substrate 51, respectively.

The above pixel openings 561, 562, 563 are disposed between any two adjacent retaining wall structures 60, and the orthographic projections of the pixel openings 561, 562, 563 on the first substrate 11 respectively cover the orthographic projections of the plurality of light-entry channels on the first substrate 11, and, the orthographic projections of the pixel openings 561, 562, 563 on the first substrate 11 respectively cover the orthographic projections of the plurality of light-emitting units 12 on the first substrate 11.

The present disclosure also provides a method for preparing a quantum dot light-emitting device, including: providing a first color quantum dot-ligand solution, where the first color quantum dot-ligand solution is a mixed solution including a first color quantum dot and a quantum dot ligand in any of the above embodiments; providing a second color quantum dot-ligand solution, where the second color quantum dot-ligand solution is a mixed solution including a second color quantum dot and a quantum dot ligand in any of the above embodiments; forming a first color sub-pixel by coating the first color quantum dot-ligand solution on the substrate and performing exposure development; forming a second color sub-pixel by coating the second color quantum dot-ligand solution on the substrate and performing exposure development.

The method for preparing the quantum dot light-emitting device provided by the present disclosure, further includes: providing a third color quantum dot-ligand solution, where the third color quantum dot-ligand solution is a mixed solution including a third color quantum dot and a quantum dot ligand in any of the above embodiments; forming a third color sub-pixel by applying the third color quantum dot-ligand solution on the substrate and performing exposure development.

The first color, the second color, and the third color in the present disclosure merely illustrate that they are different from each other, but there is no special limitation on exactly what colors they represent.

The present disclosure also provides a display device including the quantum dot light emitting device described above. The display device of the present disclosure may be an electronic device such as a cell phone, a tablet computer, a television, and the like, and will not be enumerated herein.

Example of quantum dot ligand synthesis:
(1) A quantum dot ligand

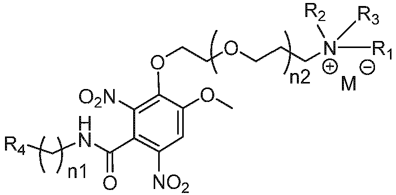

Taking R₄ as sulfhydryl, R₂ as methyl group, R₃ as ethyl group, R₁ as methyl group, M as bromine, n1 with a value of 2, and n2 with a value of 1 as an example, the synthesis route is as follows:

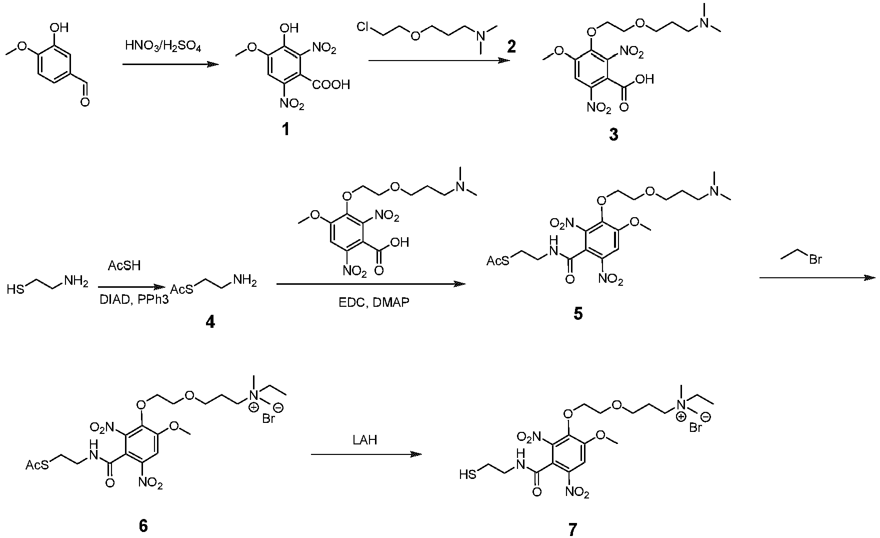

The specific synthesis method is as follows:
(a) 3-Hydroxy-4-methoxybenzaldehyde (CAS:621-59-0, 4.6g, 30mmol) as well as 10 mL of concentrated sulfuric acid are placed in a 100 mL round bottom flask and cooled using an ice bath. The concentrated nitric acid 20 mL is added drop-wise and stirred. After the drop-wise addition, stirring is continued for 1 hour under the ice bath and then this mixture is poured into ice water. After filtration, recrystallization is carried out using water to give pale yellow needle-like crystals (Compound 1).
(b) A mixture of compound 1, compound 2 with sodium hydroxide is placed in a solvent mixture of toluene and ethanol (50mL/10mL) and refluxed with stirring for 48 hours. Then the mixture is poured into water and extracted with toluene. The organic phase is washed with NaHCO3, water and saturated saline and then dried using anhydrous sodium sulfate and toluene is removed using spin distillation. The product is dissolved in a minimum amount of dichloromethane, introduced into a large amount of hexane, allowed to stand and cooled to crystallize, then filtered and dried to give compound 3.
(c) 2-Aminoethanethiol (CAS 60-23-1) is mixed with thioacetic acid (AcSH), diisopropyl azodicarboxylate (DIAD), and triphenylphosphine (PPh3) in dichloromethane, reacted for 6 hours, evaporated to dryness, and recrystallized from ethanol to give compound 4.
(d) Compound 3 is dissolved in dichloromethane and placed in an ice bath, DCC is added along with DMAP and stirred for 10 minutes at 0 degrees. Compound 4 is then added. The reaction is controlled to warm up slowly to room temperature within 2d. Filtering is performed, and the filtrate is concentrated and purified using column chromatography to give compound 5.
(e) Compound 5 is dissolved in dry dichloromethane, ethidium bromide is added and the reaction system is stirred for 2 days protected from light. The solvent and excess ethidium bromide are removed by distillation under reduced pressure. The remaining product is washed with anhydrous ether and dried to give compound 6.
(f) Compound 6 is dissolved in dry dichloromethane, placed in an ice bath, lithium aluminum hydride (LAH) is added drop-wise, the reaction is stirred and brought to room temperature over a period of 4 hours, and is extracted and dried to give compound 7, i.e., the quantum dot ligand of the present embodiment.

(2) A quantum dot ligand

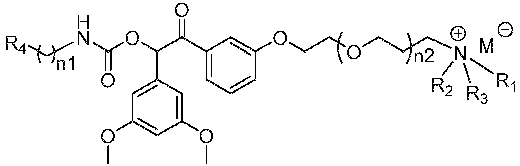

Taking R₄ as sulfhydryl, R₂ as methyl group, R₃ as methyl group, R₁ as ethyl group, M as bromine, n1 with a value of 2, and n2 with a value of 1 as an example, the synthesis route is as follows:

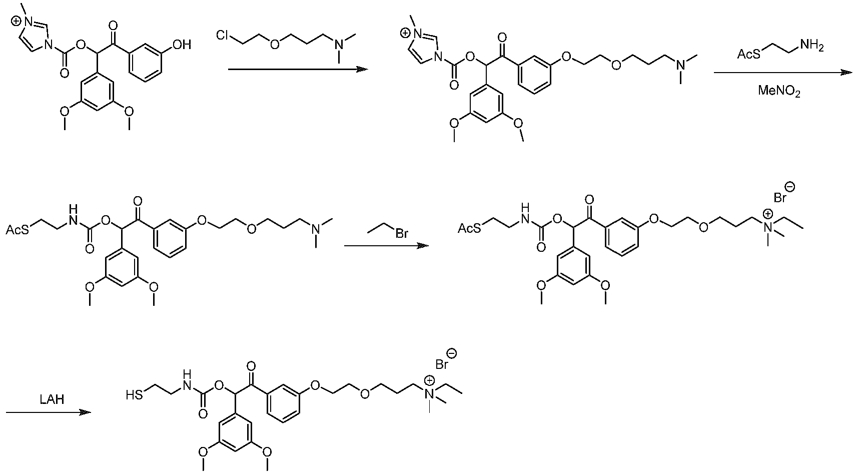

(3) A quantum dot ligand

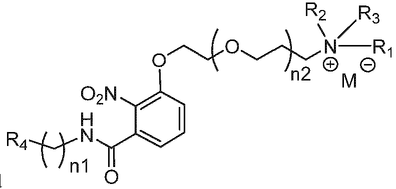

Taking R₄ as sulfhydryl, R₂ as methyl group, R₃ as ethyl group, R₁ as methyl group, M as bromine, n1 with a value of 2, and n2 with a value of 1 as an example, the synthesis route is as follows:

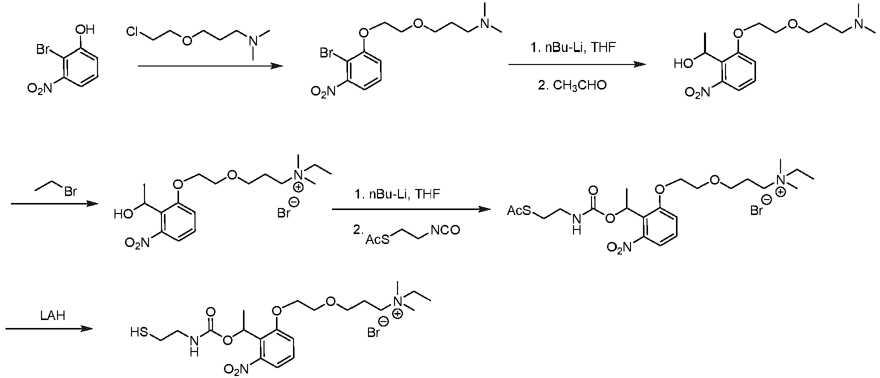

(4) A quantum dot ligand

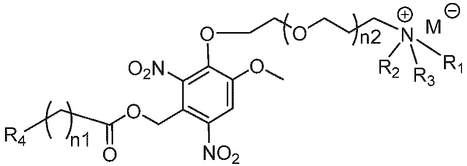

Taking R₄ as sulfhydryl, R₂ as methyl group, R₃ as ethyl group, R₁ as methyl group, M as bromine, n1 with a value of 2, and n2 with a value of 1 as an example, the synthesis route is as follows:

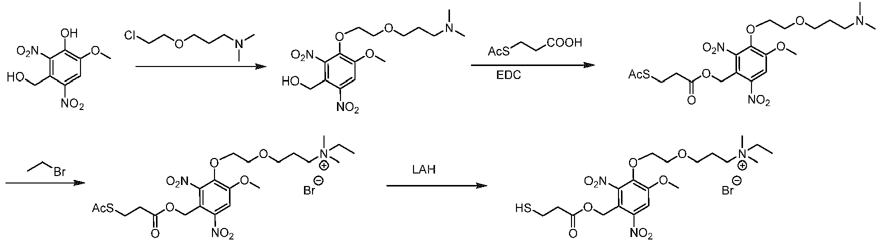

(5) A quantum dot ligand

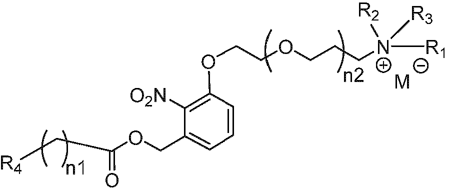

Taking R₄ as sulfhydryl, R₂ as methyl group, R₃ as ethyl group, R₁ as methyl group, M as bromine, n1 with a value of 2, and n2 with a value of 1 as an example, the synthesis route is as follows:

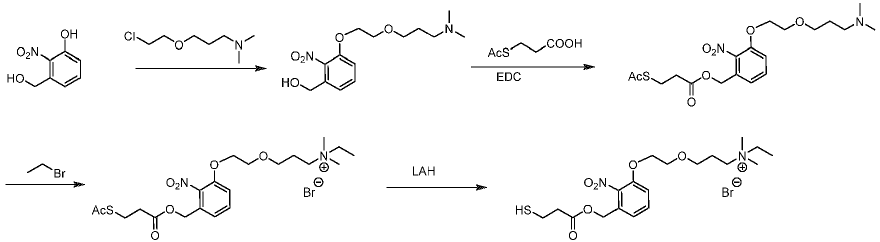

(6) A quantum dot ligand

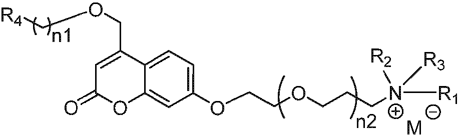

Taking R₄ as sulfhydryl, R₂ as methyl, R₃ as ethyl group, R₁ as methyl group, M as bromine, n1 with a value of 2, and n2 with a value of 1 as an example, the synthesis route is as follows:

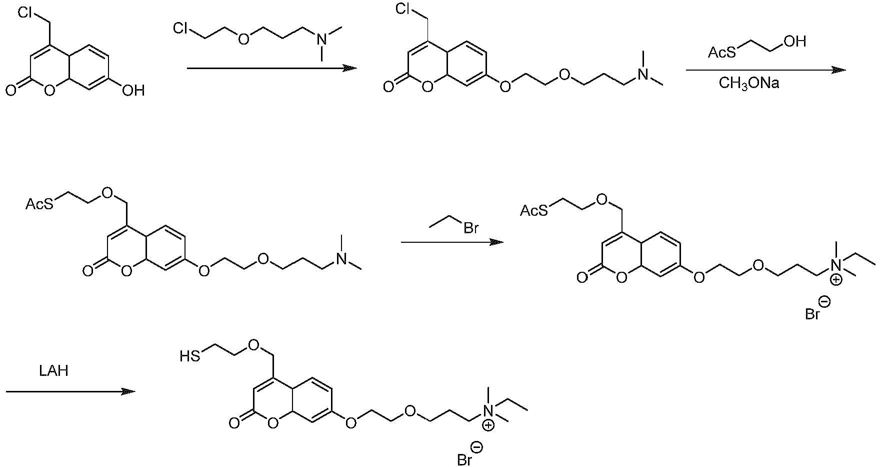

The specific method for synthesizing the quantum dot ligands (2) to (5) may refer to the quantum dot ligand (1) or other methods known to those skilled in the art.

The above quantum dot ligand synthesis formula and synthesis method are only exemplary to illustrate the synthesis method of the quantum dot ligand of the present disclosure, and those skilled in the art may synthesize other quantum dot ligands protected by the present disclosure with reference to the above method.

Photolithography process to form a quantum dot film layer
1) A quantum dot-ligand solution is provided. The quantum dot-ligand solution is a mixed solution including a quantum dot, and a quantum dot ligand. Specific steps are as follows.

The quantum dot with oleic acid ligand is added to dimethyl sulfoxide (DMSO), the DMSO solution of the quantum dot ligand provided by the present disclosure is added, the quantum dot ligand is as shown in the above embodiment, and after stirring for 4-12 hours, the quantum dot is dissolved in DMSO, indicating that the ligand exchange is complete. After precipitation/dissolution using ethyl acetate/DMSO for 3 times, the quantum dots with the quantum dot ligand of the present disclosure, i.e., the quantum dot-ligand material, are obtained by using ethyl acetate for precipitation, drying, and dissolving in ethanol.

In this step, a first color quantum dot-ligand solution, a second color quantum dot-ligand solution, and a third color quantum dot-ligand solution can be prepared by controlling the size of the quantum dots, respectively.

(2) The quantum dot-ligand solution is coated on the substrate, and an exposure treatment is carried out to cause the photosensitive group in the quantum dot-ligand material to break bonds so as to decompose the quantum dot-ligand material into a first unit including the quantum dot, the coordination group, and the connecting group, and a second unit including the dissolving group.

3) A development process is performed. The second unit is capable of being dissolved in the development solution, the first unit is not dissolved in the development solution, and the first unit forms a quantum dot film layer.

Steps 2) and 3) may specifically include the following operations:

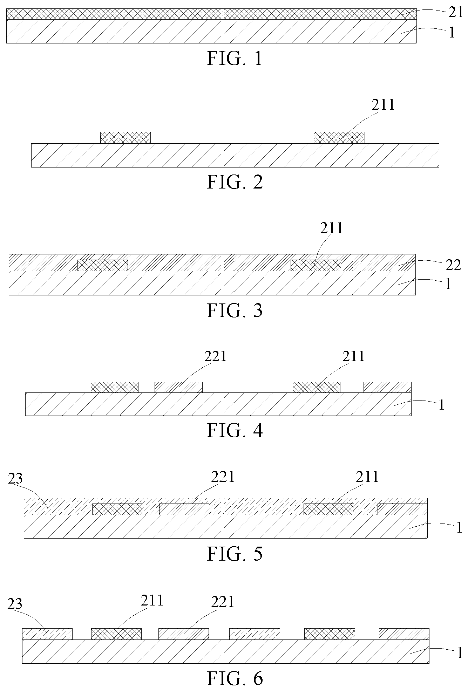

As shown in FIGS. 1 to 2, the first color quantum dot-ligand solution is coated on the substrate 1 to form the first color quantum dot film layer 21, plus a first patterning (Photo Mask) process, and the whole is exposed by using UV light; after that, the substrate is rinsed and developed by using one or more solvent mixtures of -ethanol, isopropanol, propanol, n-butanol, DMF, and DMSO as the developer; after development, the substrate is again heated at 90oC for 120 seconds to remove the developer to form the first color sub-pixel 211.

As shown in FIGS. 3 to 4, a second color quantum dot-ligand solution is coated to form a second color quantum dot film layer 22. A second Photo Mask is added, and the whole is exposed using UV light, and then the second color sub-pixel 221 is formed by developing and fixing.

As shown in FIGS. 5 to 6, a third color quantum dot-ligand solution is finally coated to form a third color quantum dot film layer 23, a third Photo Mask is added, the whole is exposed using UV light, and the third color sub-pixel 231 is formed by developing and fixing.

Takes the ligand 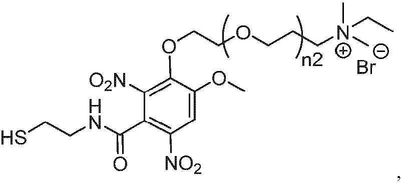 and n2 with a value of 1 as an example:

The quantum dot-ligand material formed in step 1) is:

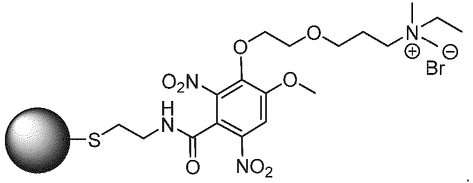

The quantum dot-ligand material reacts as follows after exposure:

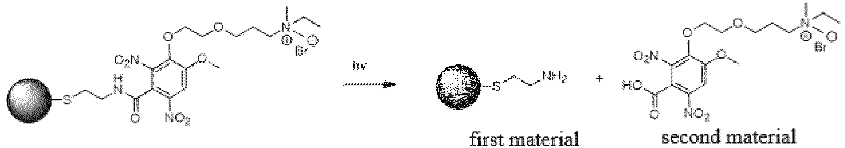

The changes in the quantum dots after exposure of the remaining ligands can be referred to above described content.

### Example of preparation of a display panel

The display panel includes a quantum dot light-emitting device, and the quantum dot light-emitting device includes an anode, a hole injection layer, a hole transport layer, a quantum dot light-emitting layer, an electron transport layer, an electron injection layer, and a cathode, which are sequentially stacked.

The display panel preparation method specifically includes the following steps:

The transparent substrate is cleaned by a standard method, and then the followings are deposited sequentially: the gate metal Mo 200 nm and patterned; the gate dielectric SiO₂ 150 nm; the active layer IGZO 40 nm and patterned; the source-drain metal Mo 200 nm and patterned; the passivation layer SiO₂ 300 nm and patterned; the pixel electrode ITO 40 nm and patterned; and finally spin-coating and deposition of acrylic-based materials are carried out, followed by photolithography and solidification to form the pixel definition layer, about 1.5µm, to form the TFT backplane.

Before preparing quantum dot light-emitting devices (QD-LEDs), the surface of the TFT backplane is treated with plasma.

The hole injection layer and hole transport layer were prepared by spin-coating process, such as spin-coating PEDOT (poly(3,4-ethylene dioxythiophene)): PSS (poly styrene sulfonic acid), and TFB, etc., respectively; their overall thicknesses are 50-100 nm.

The above-described lithography process is used to form a quantum dot film layer, specifically including, coating a first color quantum dot-ligand solution, adding a first Photo Mask, exposing the whole by UV light, and then developing and fixing to form the first color sub-pixel; then coating a second color quantum dot-ligand solution, adding a second Photo Mask, exposing the whole by UV light, and then developing and fixing to form the second color sub-pixel; and finally coating a third color quantum dot-ligand solution, adding a third Photo Mask, exposing the whole by UV light, and then developing and fixing to form the third color sub-pixel.

The electron transport layer and the electron injection layer are formed by spin coating or vapor deposition, such as ZnO nanoparticles, etc.

The thin cathode metal layer is vaporized, and the cathode can be an Al layer, etc., which is about 500-1000 nm. After vaporization, the encapsulation and cut is performed to complete the preparation of the whole display panel.

The AMQLED device may have a bottom-out light emission method, and may be prepared with a minimum sub-pixel area of 10-30 micrometers and the display panel is about 300-800 ppi.includinclud

It is noted that although the various steps of the method in the present disclosure are described in the accompanying drawings in a particular order, it is not required or implied that the steps must be performed in that particular order, or that all of the steps shown must be performed to achieve the desired results. Additional or alternatively, certain steps may be omitted, multiple steps may be combined to be performed as a single step, and/or a single step may be broken down to be performed as multiple steps, etc., all of which should be considered part of the present disclosure.

It should be appreciated that the present disclosure does not limit its application to the detailed structure and arrangement of the components presented herein. The present disclosure is capable of having other embodiments and can be realized and performed in a variety of ways. The foregoing deformed and modified forms fall within the scope of the present disclosure. It should be understood that the present disclosure, as disclosed and limited in this specification, extends to all alternative combinations of two or more individual features mentioned or apparent in the text and/or in the accompanying drawings. All of these various combinations constitute a plurality of alternative aspects of the present disclosure. The embodiments of this specification illustrate the best ways known for realizing the present disclosure and will enable those skilled in the art to utilize the present disclosure.

## Claims

1. A quantum dot ligand, wherein the quantum dot ligand has a structural general formula as shown in Formula I,
X-Y-Z Formula I;
wherein X is a coordination group for forming a coordination bond with a quantum dot body;
Z is a dissolving group, and the dissolving group is selected from a polar group;
Y comprises a linking group and a photosensitive group connected with each other, and the linking group is connected between the coordination group and the photosensitive group;
the linking group is selected from an alkylene group;
the photosensitive group is capable of undergoing bond breaking under light condition such that the quantum dot ligand decomposes into a first unit comprising the coordination group and the linking group, and a second unit comprising the dissolving group, and polarity of the first unit is less than polarity of the second unit,
wherein the photosensitive group is selected from a group comprising the following structures:

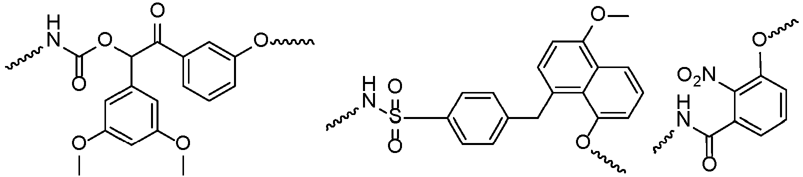

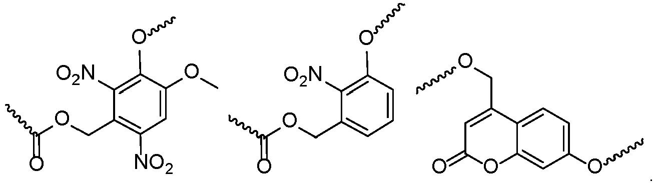

2. The quantum dot ligand according to claim 1, wherein positive and negative charge centers of the dissolving group do not overlap.

3. The quantum dot ligand according to claim 1, wherein a dielectric constant of the first unit is less than a dielectric constant of the second unit.

4. The quantum dot ligand according to claim 1, wherein the dissolving group has a dipole moment µ>0.5.

5. The quantum dot ligand according to claim 1, wherein after the photosensitive group undergoes bond breaking under the light condition, amino, hydroxyl, or carboxyl groups are formed at the end of the first unit.

6. The quantum dot ligand according to claim 1, wherein Y further comprises a solubilizing group, the solubilizing group is connected between the photosensitive group and the dissolving group, and the solubilizing group is selected from a structure comprising 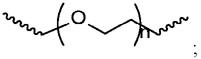
when Y further comprises the solubilizing group, the second unit further comprises the solubilizing group,
wherein n is any integer selected from 1 to 9.

7. The quantum dot ligand according to claim 1, wherein the dissolving group is selected from a quaternary ammonium salt group.

8. The quantum dot ligand according to claim 1, wherein the dissolving group is selected from a structure of 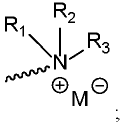
wherein R₁, R₂, and R₃ are same or different, which are independently selected from methyl group and ethyl group;
M is selected from halogen atoms.

9. The quantum dot ligand according to claim 1, wherein the quantum dot ligand is selected from a group comprising the following structures: 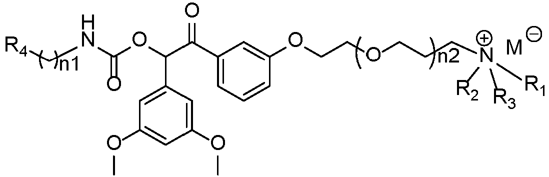 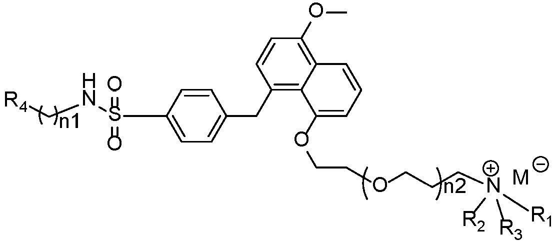 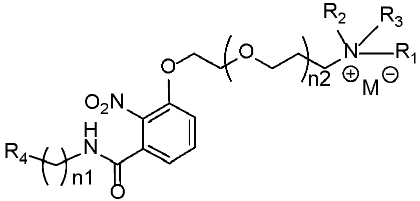 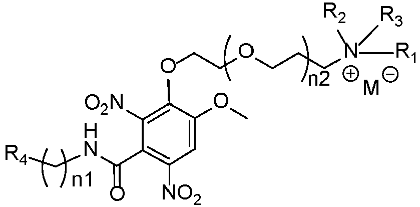 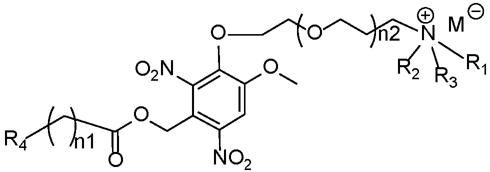 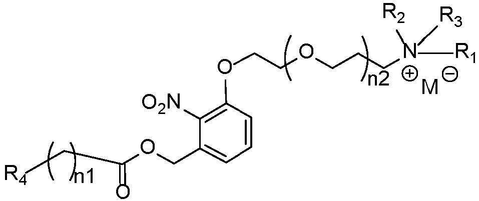 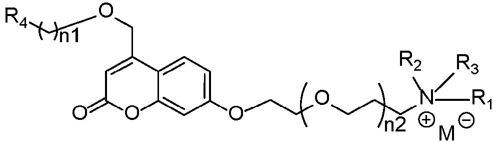
wherein R₁, R₂, and R₃ are same or different, and are independently selected from methyl group and ethyl group;
M is selected from chlorine or bromine;
R₄ is selected from amino, carboxylic acid, sulfhydryl, phosphine or phosphine oxide groups;
n1 is any integer selected from 2 to 8;
n2 is any integer selected from 1 to 9.

10. A quantum dot-ligand material, comprising the quantum dot body and the quantum dot ligand according to any one of claims 1 to 9, wherein the quantum dot ligand is connected to the quantum dot body through the coordination bond.

11. A method for preparing a quantum dot film layer (133a; 52; 21; 22; 23), comprising:
providing the quantum dot-ligand material according to claim 10;
coating the quantum dot-ligand material on a substrate (1; 11; 51), and performing an exposure treatment such that the photosensitive group in the quantum dot-ligand material undergoes bond breaking to decompose the quantum dot-ligand material into a first material comprising the quantum dot body, the coordination group, and the linking group, as well as a second material comprising the dissolving group;
performing a development process, wherein the second material is soluble in a development solution, the first material is insoluble in the development solution, and the first material forms the quantum dot film layer (133a; 52; 21; 22; 23),
preferably, wherein when the quantum dot ligand further comprises a solubilizing group, the second material further comprises the solubilizing group.

12. A method for preparing a quantum dot light-emitting device, comprising:
providing a first color quantum dot-ligand solution, wherein the first color quantum dot-ligand solution is a mixed solution comprising a first color quantum dot body and the quantum dot ligand according to any one of claims 1 to 9;
providing a second color quantum dot-ligand solution, wherein the second color quantum dot-ligand solution is a mixed solution comprising a second color quantum dot body and the quantum dot ligand according to any one of claims 1 to 9;
forming a first color sub-pixel (211) by coating the first color quantum dot-ligand solution on a substrate (1; 11; 51) and performing exposure development;
forming a second color sub-pixel (221) by coating the second color quantum dot-ligand solution on the substrate (1; 11; 51) and performing exposure development.

13. A quantum dot light-emitting device, comprising a functional layer, wherein the functional layer comprises a quantum dot film layer (133a; 52; 21; 22; 23), and the quantum dot film layer (133a; 52; 21; 22; 23) is prepared by adopting the method for preparing the quantum dot film layer according to claim 11,
preferably, wherein the quantum dot light-emitting device further comprises an anode and a cathode, and the functional layer is located between the anode and the cathode; or
preferably, wherein the quantum dot light-emitting device further comprises a light-emitting unit, with the quantum dot film layer (133a; 52; 21; 22; 23) located on one side of the light-emitting unit; or
preferably, wherein the quantum dot film layer (133a; 52; 21; 22; 23) comprises a plurality of sub-units, the sub-units comprise a first color quantum dot and a second color quantum dot, a material of the first color quantum dot comprises a first color quantum dot body and the first unit, and a material of the second color quantum dot comprises a second color quantum dot body and the quantum dot ligand in which no bond breaking occurs; and
in at least one sub-unit of the plurality of sub-units, a mass ratio of the material of the second color quantum dot to the material of the first color quantum dot is less than 10⁻³.

## Patentansprüche

1. Quantenpunktligand, wobei der Quantenpunktligand eine allgemeine Strukturformel aufweist, wie sie in Formel I gezeigt ist,
X-Y-Z Formel I;
wobei X eine Koordinationsgruppe zum Bilden einer Koordinationsbindung mit einem Quantenpunktkörper ist;
Z eine lösende Gruppe ist, und die lösende Gruppe aus einer polaren Gruppe ausgewählt ist;
Y eine verbindende Gruppe und eine lichtempfindliche Gruppe umfasst, die miteinander verbunden sind, und die verbindende Gruppe zwischen der Koordinationsgruppe und der lichtempfindlichen Gruppe verbunden ist;
die verbindende Gruppe ausgewählt ist aus einer Alkylengruppe;
die lichtempfindliche Gruppe in der Lage ist, unter Lichtbedingungen eine Bindungsbrechung zu erfahren, sodass der Quantenpunktligand in eine erste Einheit, die die Koordinationsgruppe und die verbindende Gruppe umfasst, und eine zweite Einheit, die die sich auflösende Gruppe umfasst, zerfällt, und die Polarität der ersten Einheit geringer ist als die Polarität der zweiten Einheit,
wobei die lichtempfindliche Gruppe aus einer Gruppe ausgewählt ist, umfassend die folgenden Strukturen:

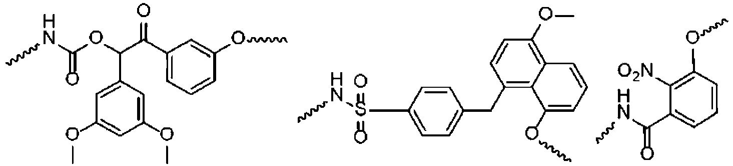

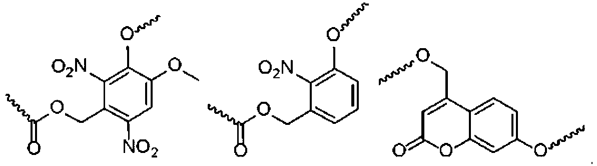

2. Quantenpunktligand nach Anspruch 1, wobei sich die positiven und negativen Ladungszentren der sich auflösenden Gruppe nicht überlappen.

3. Quantenpunktligand nach Anspruch 1, wobei eine Dielektrizitätskonstante der ersten Einheit kleiner ist als eine Dielektrizitätskonstante der zweiten Einheit.

4. Quantenpunktligand nach Anspruch 1, wobei die sich auflösende Gruppe ein Dipolmoment µ>0,5 aufweist.

5. Quantenpunktligand nach Anspruch 1, wobei nach der Bindungsbrechung der lichtempfindlichen Gruppe unter den Lichtbedingungen Amino-, Hydroxyl- oder Carboxylgruppen am Ende der ersten Einheit gebildet werden.

6. Quantenpunktligand nach Anspruch 1, wobei Y ferner eine solubilisierende Gruppe umfasst, die solubilisierende Gruppe zwischen der lichtempfindlichen Gruppe und der lösenden Gruppe verbunden ist und die solubilisierende Gruppe aus einer Struktur ausgewählt ist, umfassend 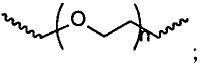
wenn Y ferner die solubilisierende Gruppe umfasst, umfasst die zweite Einheit ferner die solubilisierende Gruppe;
wobei n eine beliebige ganze Zahl von 1 bis 9 ist.

7. Quantenpunktligand nach Anspruch 1, wobei die auflösende Gruppe aus einer quaternären Ammoniumsalzgruppe ausgewählt ist.

8. Quantenpunktligand nach Anspruch 1, wobei die auflösende Gruppe ausgewählt ist aus einer Struktur von 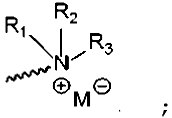
wobei R₁, R₂ und R₃ gleich oder unterschiedlich sind, die unabhängig voneinander aus einer Methylgruppe und einer Ethylgruppe ausgewählt sind;
M ausgewählt ist aus Halogenatomen.

9. Quantenpunktligand nach Anspruch 1, wobei der Quantenpunktligand aus einer Gruppe ausgewählt ist, umfassend die folgenden Strukturen: 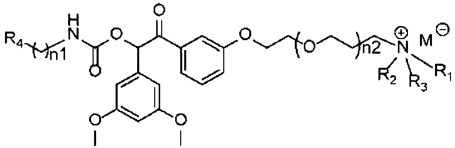 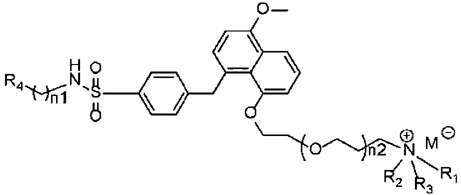 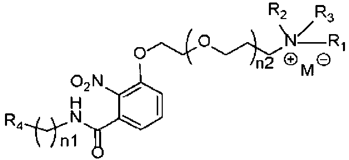 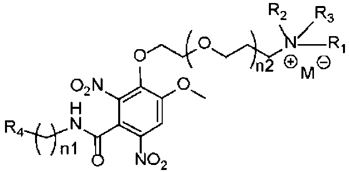 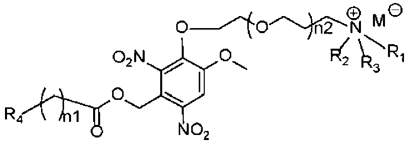 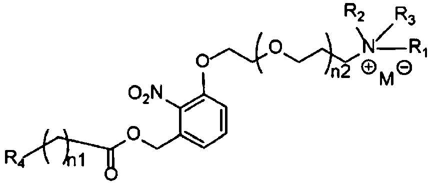 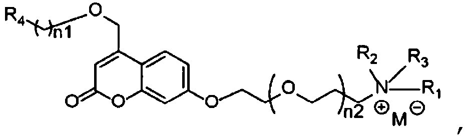
wobei R₁, R₂ und R₃ gleich oder unterschiedlich sind und unabhängig voneinander aus einer Methylgruppe und einer Ethylgruppe ausgewählt sind;
M ausgewählt ist aus Chlor oder Brom;
R₄ ausgewählt ist aus Amino-, Carbonsäure-, Sulfhydryl-, Phosphin- oder Phosphinoxidgruppen;
n1 eine beliebige ganze Zahl von 2 bis 8 ist;
n2 eine beliebige ganze Zahl von 1 bis 9 ist.

10. Quantenpunktligandenmaterial, umfassend den Quantenpunktkörper und den Quantenpunktliganden nach einem der Ansprüche 1 bis 9, wobei der Quantenpunktligand über die Koordinationsbindung mit dem Quantenpunktkörper verbunden ist.

11. Verfahren zur Herstellung einer Quantenpunktfilmschicht (133a; 52; 21; 22; 23), umfassend:
Bereitstellen des Quantenpunktligandenmaterials nach Anspruch 10;
Beschichten des Quantenpunktligandenmaterials auf ein Substrat (1; 11; 51) und Durchführen einer Belichtungsbehandlung, sodass die lichtempfindliche Gruppe in dem Quantenpunktligandenmaterial eine Bindungsbrechung erfährt, um das Quantenpunktligandenmaterial in ein erstes Material, das den Quantenpunktkörper, die Koordinationsgruppe und die Verbindungsgruppe umfasst, sowie in ein zweites Material, das die sich auflösende Gruppe umfasst, zu zerlegen;
Durchführen eines Entwicklungsprozesses, wobei das zweite Material in einer Entwicklungslösung löslich ist, das erste Material in der Entwicklungslösung unlöslich ist, und das erste Material die Quantenpunktfilmschicht (133a; 52; 21; 22; 23) bildet,
vorzugsweise, wobei, wenn der Quantenpunktligand ferner eine solubilisierende Gruppe umfasst, das zweite Material ferner die solubilisierende Gruppe umfasst.

12. Verfahren zur Herstellung einer lichtemittierenden Quantenpunktvorrichtung, umfassend:
Bereitstellen einer ersten Farbquantenpunktligandenlösung, wobei die erste Farbquantenpunktligandenlösung eine gemischte Lösung ist, die einen ersten Farbquantenpunktkörper und den Quantenpunktliganden nach einem der Ansprüche 1 bis 9 umfasst;
Bereitstellen einer zweiten Farbquantenpunktligandenlösung, wobei die zweite Farbquantenpunktligandenlösung eine gemischte Lösung ist, die einen zweiten Farbquantenpunktkörper und den Quantenpunktliganden nach einem der Ansprüche 1 bis 9 umfasst;
Bilden eines ersten Farbunterpixels (211) durch Auftragen der ersten Farbquantenpunktligandenlösung auf ein Substrat (1; 11; 51) und Durchführen einer Belichtungsentwicklung;
Bilden eines zweiten Farbunterpixels (221) durch Auftragen der zweiten Farbquantenpunktligandenlösung auf das Substrat (1; 11; 51) und Durchführen einer Belichtungsentwicklung.

13. Lichtemittierende Quantenpunktvorrichtung, umfassend eine funktionelle Schicht, wobei die funktionelle Schicht eine Quantenpunktfilmschicht (133a; 52; 21; 22; 23) umfasst, und die Quantenpunktfilmschicht (133a; 52; 21; 22; 23) durch Anwendung des Verfahrens zur Herstellung der Quantenpunktfilmschicht nach Anspruch 11 hergestellt wird,
vorzugsweise, wobei die lichtemittierende Quantenpunktvorrichtung ferner eine Anode und eine Kathode umfasst und sich die funktionelle Schicht zwischen der Anode und der Kathode befindet; oder
vorzugsweise, wobei die lichtemittierende Quantenpunktvorrichtung ferner eine lichtemittierende Einheit umfasst, wobei sich die Quantenpunktfilmschicht (133a; 52; 21; 22; 23) auf einer Seite der lichtemittierenden Einheit befindet; oder
vorzugsweise, wobei die Quantenpunktfilmschicht (133a; 52; 21; 22; 23) eine Vielzahl von Untereinheiten umfasst, die Untereinheiten einen ersten Farbquantenpunkt und einen zweiten Farbquantenpunkt umfassen, ein Material des ersten Farbquantenpunkts einen ersten Farbquantenpunktkörper und die erste Einheit umfasst, und ein Material des zweiten Farbquantenpunkts einen zweiten Farbquantenpunktkörper und den Quantenpunktliganden umfasst, in dem keine Bindungsbrechung stattfindet; und
in mindestens einer Untereinheit der Vielzahl von Untereinheiten ein Massenverhältnis des Materials des zweiten Farbquantenpunkts zu dem Material des ersten Farbquantenpunkts weniger als 10⁻³ beträgt.

## Revendications

1. Ligand à points quantiques, dans lequel le ligand à points quantiques a une formule générale structurale comme indiqué dans la formule I,
Formule I X-Y-Z ;
dans lequel X est un groupe de coordination destiné à former un lien de coordination avec un corps de points quantiques ;
Z est un groupe dissolvant, et le groupe dissolvant est choisi parmi un groupe polaire ;
Y comprend un groupe de liaison et un groupe photosensible reliés l'un à l'autre, et le groupe de liaison est relié entre le groupe de coordination et le groupe photosensible ;
le groupe de liaison est choisi parmi un groupe alkylène ;
le groupe photosensible est capable de subir une rupture de lien sous l'effet de la lumière de sorte que le ligand à points quantiques se décompose en une première unité comprenant le groupe de coordination et le groupe de liaison, et une seconde unité comprenant le groupe dissolvant, et la polarité de la première unité est inférieure à la polarité de la seconde unité,
dans lequel le groupe photosensible est choisi parmi un groupe comprenant les structures suivantes :

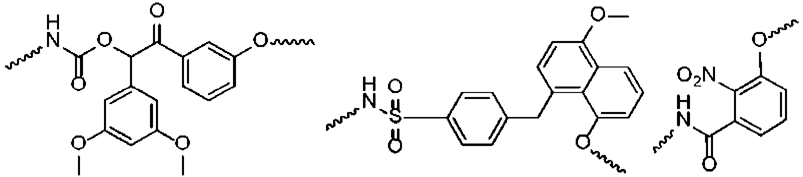

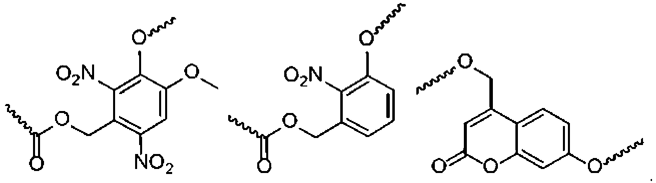

2. Ligand à points quantiques selon la revendication 1, dans lequel les centres de charge positifs et négatifs du groupe dissolvant ne se chevauchent pas.

3. Ligand à points quantiques selon la revendication 1, dans lequel une constante diélectrique de la première unité est inférieure à la constante diélectrique de la seconde unité.

4. Ligand à points quantiques selon la revendication 1, dans lequel le groupe dissolvant a un moment dipolaire µ>0,5.

5. Ligand à points quantiques selon la revendication 1, dans lequel après que le groupe photosensible subit une rupture de lien sous l'effet de la lumière, des groupes amino, hydroxyle, ou carboxyle sont formés à l'extrémité de la première unité.

6. Ligand à points quantiques selon la revendication 1, dans lequel Y comprend en outre un groupe solubilisant, le groupe solubilisant est relié entre le groupe photosensible et le groupe dissolvant, et le groupe solubilisant est choisi parmi une structure comprenant 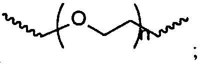
lorsque Y comprend en outre le groupe solubilisant, la seconde unité comprend en outre le groupe solubilisant ;
dans lequel n est un entier quelconque choisi entre 1 et 9.

7. Ligand à points quantiques selon la revendication 1, dans lequel le groupe dissolvant est choisi parmi un groupe de sel d'ammonium quaternaire.

8. Ligand à points quantiques selon la revendication 1, dans lequel le groupe dissolvant est choisi parmi une structure de 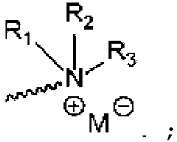
dans lequel R₁, R₂, et R₃ sont identiques ou différents, qui sont sélectionnés indépendamment parmi les groupes méthyle et éthyle ;
M est choisi parmi les atomes d'halogène.

9. Ligand à points quantiques selon la revendication 1, dans lequel le ligand à points quantiques est choisi parmi un groupe comprenant les structures suivantes : 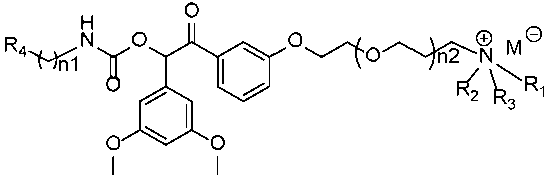 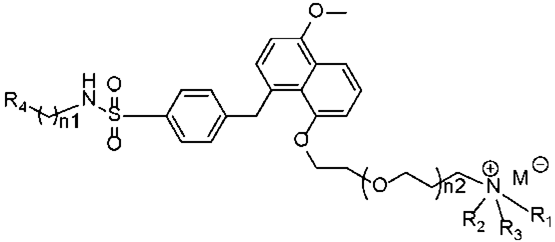 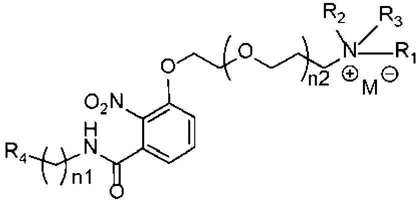 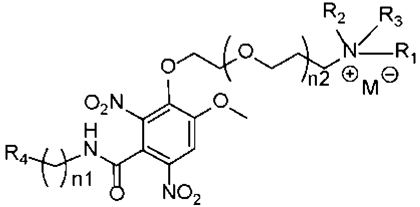 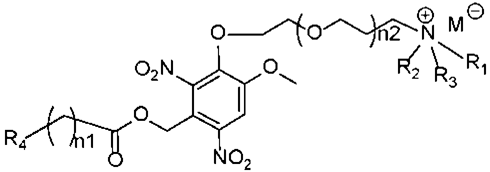 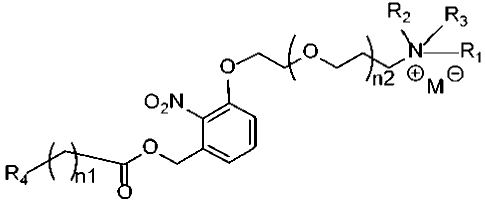 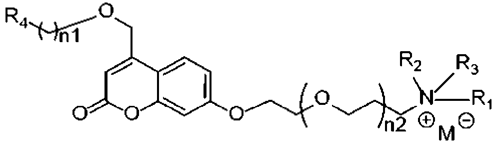
dans lequel R₁, R₂, et R₃ sont identiques ou différents, et sont sélectionnés indépendamment parmi les groupes méthyle et éthyle ;
M est choisi parmi le chlore ou le brome ;
R₄ est choisi parmi les groupes amino, acide carboxylique, sulfhydryle, phosphine ou oxyde de phosphine ;
n1 est un entier quelconque sélectionné entre 2 et 8 ;
n2 est un entier quelconque choisi entre 1 et 9.

10. Matériau de ligand à points quantiques, comprenant le corps de points quantiques et le ligand à points quantiques selon l'une quelconque des revendications 1 à 9, dans lequel le ligand à points quantiques est relié au corps de points quantiques par le lien de coordination.

11. Procédé de préparation d'une couche de film de points quantiques (133a ; 52 ; 21 ; 22 ; 23) comprenant :
la fourniture du matériau de ligand à points quantiques selon la revendication 10 ;
le revêtement du matériau de ligand à points quantiques sur un substrat (1 ; 11 ; 51), et la réalisation d'un traitement d'exposition de sorte que le groupe photosensible du matériau de ligand à points quantiques subit une rupture de lien pour décomposer le matériau de ligand à points quantiques en un premier matériau comprenant le corps de points quantiques, le groupe de coordination, et le groupe de liaison, ainsi qu'un deuxième matériau comprenant le groupe de dissolution ;
la réalisation d'un processus de développement, dans lequel le deuxième matériau est soluble dans une solution de développement, le premier matériau est insoluble dans la solution de développement, et le premier matériau forme la couche de film à points quantiques (133a ; 52 ; 21 ; 22 ; 23),
de préférence, lorsque le ligand à points quantiques comprend en outre un groupe solubilisant, le second matériau comprend en outre le groupe solubilisant.

12. Procédé de préparation d'un dispositif électroluminescent à points quantiques, comprenant :
la fourniture d'une première solution de ligand à points quantiques de couleur, dans lequel la première solution de ligand à points quantiques de couleur est une solution mixte comprenant un corps de points quantiques de première couleur et le ligand à points quantiques selon l'une quelconque des revendications 1 à 9 ;
la fourniture d'une solution de ligand à points quantiques de seconde couleur, dans lequel la solution de ligand à points quantiques de seconde couleur est une solution mixte comprenant un corps de points quantiques de seconde couleur et le ligand à points quantiques selon l'une quelconque des revendications 1 à 9 ;
la formation d'un premier sous-pixel de couleur (211) en déposant la première solution de ligand à points quantiques de couleur sur un substrat (1 ; 11 ; 51) et en réalisant le développement de l'exposition ;
la formation d'un second sous-pixel de couleur (221) en déposant la solution de ligand à points quantiques de seconde couleur sur le substrat (1 ; 11 ; 51) et en réalisant le développement de l'exposition.

13. Dispositif électroluminescent à points quantiques, comprenant une couche fonctionnelle, dans lequel la couche fonctionnelle comprend une couche de film à points quantiques (133a ; 52 ; 21 ; 22 ; 23), et la couche de film à points quantiques (133a ; 52 ; 21 ; 22 ; 23) est préparé en adoptant le procédé de préparation de la couche de film à points quantiques selon la revendication 11,
de préférence, dans lequel le dispositif électroluminescent à points quantiques comprend en outre une anode et une cathode, et la couche fonctionnelle est située entre l'anode et la cathode ; ou
de préférence, dans lequel le dispositif électroluminescent à points quantiques comprend en outre une unité électroluminescente, avec la couche de film à points quantiques (133a ; 52 ; 21 ; 22 ; 23) situé sur un côté de l'unité émettrice de lumière ; ou
de préférence, dans lequel la couche de film à points quantiques (133a ; 52 ; 21 ; 22 ; 23) comprend une pluralité de sous-unités, les sous-unités comprennent un premier point quantique de couleur et un deuxième point quantique de couleur, un matériau du premier point quantique de couleur comprend un corps de points quantiques de première couleur et la première unité, et un matériau du second point quantique de couleur comprend un second corps de points quantiques de seconde couleur et le ligand à points quantiques dans lequel aucune rupture de lien ne se produit ; et
dans au moins une sous-unité de la pluralité de sous-unités, un rapport de masse du matériau du second point quantique de couleur au matériau du premier point quantique de couleur est inférieur à 10⁻³.
